# EUROPÄISCHE PATENTSCHRIFT

(11) **EP 3 178 547 B1**
(45) Veröffentlichungstag und Bekanntmachung des Hinweises auf die Patenterteilung: **13.06.2018**
(21) Anmeldenummer: 16202448.3
(22) Anmeldetag: 06.12.2016
(51) Int. Cl.: B01F 11/00, B01F 15/02, B01F 13/06, B01F 13/00, B01F 15/00

(54) **VAKUUMMISCHVORRICHTUNG MIT BEDIENELEMENT, DRUCK- UND VAKUUMPUMPE ZUM MISCHEN VON POLYMETHYLMETHACRYLAT-KNOCHENZEMENT, UND VERFAHREN**
VACUUM MIXING DEVICE WITH OPERATING ELEMENT, PRESSURE PUMP, AND VACUUM PUMP FOR MIXING POLYMETHYLMETHACRYLATE BONE CEMENT, AND PROCESS
DISPOSITIF DE MÉLANGE SOUS VIDE COMPRENANT UN ÉLÉMENT DE COMMANDE, POMPE DE REFOULEMENT ET À VIDE DESTINÉE À MÉLANGER LE CIMENT OSSEUX EN POLYMÉTHACRYLATE DE MÉTHYLE, ET PROCÉDÉ

(30) Priorität: 07.12.2015 DE 102015121277
(43) Veröffentlichungstag der Anmeldung: 14.06.2017
(73) Patentinhaber: Heraeus Medical GmbH, 61273 Wehrheim (DE)
(72) Erfinder: Kluge, Thomas Dr., 56179 Vallendar (DE); Vogt, Sebastian Dr., 99092 Erfurt (DE)
(74) Vertreter: Schultheiss & Sterzel Patentanwälte PartG mbB

(56) Entgegenhaltungen:
- EP-A1- 0 882 436
- WO-A1-2010/139078
- DE-B3-102014 108 569
- DE-U1- 20 005 333
- US-A- 5 505 538

## Beschreibung

Die Erfindung betrifft eine Vakuummischvorrichtung zum Mischen von Polymethylmethacrylat-Knochenzement (PMMA-Knochenzement) aus zwei Ausgangskomponenten, insbesondere zum Mischen eines medizinischen Knochenzements, und zur Lagerung der Ausgangskomponenten. Die Erfindung betrifft ferner ein Verfahren zum Mischen von Polymethylmethacrylat-Knochenzement.

Gegenstand der Erfindung ist somit eine Vakuummischvorrichtung für die Lagerung, Vermischung und gegebenenfalls auch den Austrag von Polymethylmethacrylat-Knochenzement. Weiterhin betrifft die Erfindung ein Verfahren zum Transfer von Monomerflüssigkeit in die Vakuummischvorrichtung und ein Verfahren zum Vermischen der Komponenten von Polymethylmethacrlyat-Knochenzement unter Vakuum beziehungsweise bei Unterdruck.

Polymethylmethacrylat-Knochenzemente (PMMA-Knochenzemente) gehen auf die grundlegenden Arbeiten von Sir Charnley zurück. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind. Zudem weist das Knochenzementpulver einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Zementteigs, bis dieser erstarrt.

Das am häufigsten in Polymethylmethacrylat-Knochenzementen verwendete Monomer ist Methylmethacrylat. Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Bestandteile der Redoxinitiatorsysteme zusammenwirken. Deshalb werden die Bestandteile des Redoxinitiatorsystems in den separaten Ausgangskomponenten so angeordnet, dass diese keine radikalische Polymerisation auslösen können. Die Ausgangskomponenten sind bei geeigneter Zusammensetzung lagerstabil. Erst bei Vermischung der beiden Ausgangskomponenten zu einem Zementteig reagieren die zuvor getrennt in den beiden Pasten, Flüssigkeiten oder Pulvern gelagerten Bestandteile des Redoxinitiatorsystems, wobei Radikale gebildet werden, welche die radikalische Polymerisation des wenigstens einen Monomers auslösen. Die radikalische Polymerisation führt dann unter Verbrauch des Monomers zu Bildung von Polymeren, wobei der Zementteig aushärtet.

PMMA-Knochenzemente können in geeigneten Mischbechern mit Hilfe von Spateln durch Vermischen des Zementpulvers mit der Monomerflüssigkeit vermischt werden. Nachteilig ist an dieser Vorgehensweise, dass Lufteinschlüsse im gebildeten Zementteig vorhanden sein können, die später eine mechanische Destabilisierung des Knochenzements verursachen können.

Aus diesem Grund wird das Vermischen von Knochenzementpulver mit der Monomerflüssigkeit in Mischvorrichtungen mit Vakuumquellen bevorzugt, weil durch Mischen im Vakuum Lufteinschlüsse aus dem Zementteig weitgehend entfernt werden und damit eine optimale Zementqualität erreicht wird. Im Vakuum, beziehungsweise bei Unterdruck gemischte Knochenzemente haben eine deutlich verringerte Porosität und zeigen daher verbesserte mechanische Eigenschaften. Es wurden eine Vielzahl von Vakuum-Zementiersystemen offen gelegt, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, WO 00/35506 A1, EP 1 005 901 A2, US 5 344 232 A. Bei den dargelegten Vakuum-Zementiersystemen ist es erforderlich, zur Erzeugung des Unterdrucks eine externe Vakuumpumpe anzuschließen. Diese werden im Allgemeinen mit Druckluft unter Nutzung des Venturi-Prinzips betrieben. Die für den Betrieb der Vakuumpumpen notwendige Druckluft wird entweder aus stationären Druckluftanlagen oder auch aus elektrisch betriebenen Kompressoren bezogen. Daneben ist es auch möglich, für die Vakuumerzeugung elektrisch betriebene Vakuumpumpen zu verwenden.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche geschlossenen Full-Prepacked-Mischsysteme wurden mit der EP 0 692 229 A1, der DE 10 2009 031 178 B3, der US 5 997 544 A, der US 6 709 149 B1, der DE 698 12 726 T2 und der US 5 588 745 A vorgeschlagen. Auch bei diesen Mischsystemen ist eine externe Vakuumquelle notwendig. Das Patent DE 10 2009 031 178 B3 offenbart dabei eine Vakuum-Mischvorrichtung mit einem zweiteiligen Austragskolben, der auch für eine erfindungsgemäße Vakuummischvorrichtung einsetzbar ist. Dabei wird eine Kombination aus einem Gas-durchlässigen Sterilisationskolben und einem Gas-undurchlässigen Dichtungskolben verwendet.

Bei der Verwendung von Vakuummischvorrichtungen zur Zementierung müssen externe Vakuumpumpen beigestellt werden. Diese Vakuumpumpen sind kostenintensiv und müssen nach der Anwendung gereinigt werden. Weiterhin sind Vakuumschläuche zur Verbindung der Vakuumpumpen mit den Vakuummischvorrichtungen notwendig. Diese Vakuumschläuche müssen den Vakuummischvorrichtungen beigelegt sein. Vor dem Mischen mit einer Vakuummischvorrichtung muss daher zuerst die Vakuumpumpe im Operations-Saal (OP-Saal) aufgebaut und an eine Energiequelle, wie beispielsweise Druckluft, oder an eine elektrische Spannungsquelle angeschlossen werden. Danach wird die Vakuumpumpe mit einem Vakuumschlauch mit der Vakuummischvorrichtung verbunden. Diese Montageschritte kosten wertvolle OP-Zeit und sind potentiell fehlerbehaftet. Die Vakuumpumpe und die Verbindungsleitungen zur Vakuummischvorrichtung und zu externen Energiequellen und Versorgungsleitungen benötigen Platz und stellen potentielle Stolperfallen und Hindernisse dar, die den gelegentlich hektischen Ablauf während einer Operation stören können.

Ein weiteres interessantes Konzept wird mit der EP 1 886 647 A1 vorgeschlagen. Das Zementpulver ist dabei in einer evakuierten Kartusche gelagert und die Monomerflüssigkeit befindet sich in einem separaten Behälter. Beim Öffnen der unter Unterdruck stehenden Kartusche wird die Monomerflüssigkeit in die Kartusche gesaugt ohne dass Luft einströmt. Es entsteht ein von Lufteinschlüssen freier Knochenzementteig. Dieses Konzept erfordert es, dass die Kartusche während der Lagerung vor ihrer Verwendung vakuumdicht verschlossen bleibt und keine unsterile Luft eindringen kann. Die Kartusche muss dazu stabil hermetisch abgedichtet sein. Nachteilig ist hieran also, dass der Aufbau aufwendig ist und dass der Inhalt der Kartusche nach dem Einsaugen der Monomerflüssigkeit nicht von einem extern zu bedienenden Mischsystem durchmischt werden kann, da eine Durchführung für einen Mischstab oder ein Mischrohr nicht ohne weiteres dauerhaft vakuumdicht ist.

Auch die DE 200 05 333 U1 offenbart eine Vakuummischvorrichtung und ein Verfahren zur Vermischung von PMMA-Knochenzement.

Die Aufgabe der Erfindung besteht also darin, die Nachteile des Stands der Technik zu überwinden. Insbesondere sollen die Nachteile der bekannten Vakuummischvorrichtungen als Full-Prepacked-Systeme mit externer Vakuumquelle überwunden werden, ohne dass ein Unterdruck über lange Zeit gehalten werden muss. Die Erfindung hat insbesondere die Aufgabe eine Vakuummischvorrichtung zu entwickeln, bei der erst unmittelbar vor dem Vermischen der Zementkomponenten ein Unterdruck erzeugt wird. Die Vorrichtung soll maximal vereinfacht sein und es erlauben, zumindest einmalig einen Unterdruck in einer Zementkartusche gegenüber der umgebenden Atmosphäre zu erzeugen. Weiterhin kann es vorteilhaft sein, wenn die Vakuummischvorrichtung in der Lage ist, einen Transfer von Monomerflüssigkeit aus einem Monomerbehälter in eine mit Zementpulver gefüllte Kartusche zu ermöglichen. Es soll dann ferner ein Verfahren bereitgestellt werden, das einen Monomertransfer und ein Vakuummischen in Full-Prepacked-Mischvorrichtungen ermöglicht. Weiterhin soll die zu entwickelnde Vakuummischvorrichtung hauptsächlich aus kostengünstigem Kunststoff gefertigt werden können.

Die Erfindung hat ferner die Aufgabe, eine einfache geschlossene Prepack-Mischvorrichtung zu entwickeln, bei der Polymethylmethacrylat-Knochenzementpulver und Monomerflüssigkeit in separaten Kompartimenten gelagert und anschließend vermischt werden können. Unmittelbar vor der Vermischung der Komponenten soll die Monomerflüssigkeit ohne Nutzung von externen Vakuumquellen, externen elektrischen Antrieben und externen Druckluftantieben in das Zementpulver transferiert werden. Mit der Prepack-Mischvorrichtung soll unabhängig von zusätzlichen externen Vorrichtungen rein durch manuelle Betätigung Polymethylmethacrylat-Knochenzement erzeugt werden können. Dabei soll die manuelle Betätigung so weit wie möglich vereinfacht werden. Vorzugsweise soll das Öffnen der Monomerampulle, beziehungsweise der Monomerampullen, der Monomertransfer, die Erzeugung eines Vakuums oder eines Unterdrucks und die Vermischung der Zementkomponenten nur durch möglichst eine einfache Bewegung bewirkt werden, die besonders bevorzugt nur wenige Male, beispielsweise 3 bis 5 mal, wiederholt werden muss. Dadurch soll die Benutzung der Vakuummischvorrichtung für den Anwender maximal vereinfacht werden, so dass kostenintensive Schulungen eingeschränkt beziehungsweise eingespart werden können. Weiterhin soll durch eine maximal vereinfachte Bedienung der Vakuummischvorrichtung mögliche Bedienungsfehler minimiert werden, wodurch die Patientensicherheit erhöht wird.

Vom medizinischen Anwender soll das Polymethylmethacrylat-Knochenzementpulver mit der Monomerflüssigkeit innerhalb der Vakuummischvorrichtung zusammengeführt und vermischt werden können, ohne dass beide Zementkomponenten mit dem medizinischen Anwender in Berührung kommen. Ein Kontakt des medizinischen Anwenders mit dem Polymethylmethacrylat-Knochenzementpulver und mit der Monomerflüssigkeit muss ausgeschlossen sein. Bei der zu entwickelnden Vakuummischvorrichtung handelt es sich um eine Full-Prepack Vakuummischvorrichtung. Die Vakuummischvorrichtung soll so beschaffen sein, dass ein Transfer der Monomerflüssigkeit in das Polymethylmethacrylat-Knochenzementpulver durch Vakuum ohne die Verwendung von externen Vakuumpumpen erfolgt. Des Weiteren soll die Vakuummischvorrichtung ohne externe Energiequellen, wie Druckluft, Vakuum oder elektrischer Strom, auch unter einfachsten äußeren Bedingungen funktionsfähig und zuverlässig die Herstellung von Knochenzementteig gewährleisten. Besonders bevorzugt soll die Vakuummischvorrichtung möglichst weitgehend auch ohne interne Energiespeicher, wie beispielsweise Batterien oder auch mechanische Energiespeicher auskommen Die Vakuummischvorrichtung soll ohne zusätzliche technische Ausrüstung autonom verwendungsfähig sein.

Des Weiteren soll eine kostengünstig zu fertigende und zuverlässig funktionierende Vakuummischvorrichtung zum Mischen eines medizinischen Zements und gegebenenfalls zur Lagerung der Ausgangskomponenten sowie ein Verfahren zum Mischen des Knochenzements gefunden werden, bei dem eine möglichst einfache manuelle Bedienung zum Mischen der Ausgangskomponenten angewendet werden kann, möglichst ohne dass Lufteinschlüsse in dem Mischgut entstehen können.

Die Hauptkomponente des Polymethylmethacrylat-Knochenzements als Mischgut soll ein Pulver sein und die zweite Komponente soll in Form einer Flüssigkeit vorliegen. Die beiden Ausgangskomponenten des Knochenzements sollen bevorzugt in der Vakuummischvorrichtung getrennt gelagert werden können und durch Anwendung der Vorrichtung sicher zusammengeführt werden können.

Die Aufgaben der Erfindung werden gelöst durch eine Vakuummischvorrichtung zum Mischen von Polymethylmethacrylat-Knochenzement aus einer Monomerflüssigkeit und einem Zementpulver, die Vakuummischvorrichtung aufweisend zumindest eine Kartusche umfassend einen evakuierbaren Innenraum zum Mischen des Knochenzements,
eine Mischeinrichtung zum Durchmischen des Inhalts des Innenraums der zumindest einen Kartusche, die beweglich im Innenraum angeordnet ist,
eine Aufnahme zur Aufnahme eines die Monomerflüssigkeit enthaltenden separaten Behälters oder aufweisend einen integrierten Behälter enthaltend die Monomerflüssigkeit,
eine Öffnungseinrichtung, die im Bereich der Aufnahme gegen die Aufnahme beweglich angeordnet ist, so dass durch eine Bewegung der Öffnungseinrichtung ein in der Aufnahme angeordneter separater Behälter mit der Öffnungseinrichtung zu öffnen ist, oder die Öffnungseinrichtung im Bereich des integrierten Behälters gegen den integrierten Behälter beweglich angeordnet ist, so dass durch eine Bewegung der Öffnungseinrichtung der integrierte Behälter mit der Öffnungseinrichtung zu öffnen ist,
eine Vakuumpumpe, in der ein beweglicher Vakuumkolben zum Erzeugen eines Unterdrucks angeordnet ist und der einen Vakuumpumpraum der Vakuumpumpe begrenzt,
eine Druckpumpe, in der ein beweglicher Pumpkolben zum Fördern einer Flüssigkeit angeordnet ist und der einen Druckpumpraum der Druckpumpe begrenzt,
eine Verbindungsleitung, die den Innenraum der zumindest einen Kartusche mit dem Vakuumpumpraum der Vakuumpumpe verbindet, und
eine Fluidverbindung, die den Innenraum der zumindest einen Kartusche mit dem Druckpumpraum der Druckpumpe verbindet, wobei
die Vakuummischvorrichtung ein von außen bedienbares Bedienelement aufweist, wobei mit dem Bedienelement der Vakuumkolben in der Vakuumpumpe manuell bewegbar ist,
mit demselben Bedienelement der Pumpkolben in der Druckpumpe manuell bewegbar ist,
mit demselben Bedienelement die Öffnungseinrichtung gegen die Aufnahme oder gegen den integrierten Behälter zu bewegen ist, und wobei
mit demselben Bedienelement die Mischeinrichtung im Innenraum der Kartusche zum Durchmischen des Inhalts des Innenraums der Kartusche bewegbar ist.

Bevorzugt ist die Vakuummischvorrichtung auch zum Lagern der Ausgangskomponenten des Polymethylmethacrylat-Knochenzements geeignet. Besonders bevorzugt sind die Monomerflüssigkeit und/oder das Zementpulver in der Vakuummischvorrichtung enthalten. Bei den Ausgangskomponenten des Polymethylmethacrylat-Knochenzements handelt es sich um das Zementpulver und die Monomerflüssigkeit, wobei die Monomerflüssigkeit bevorzugt in einer Glasampulle enthalten ist, die als separater Behälter in der Aufnahme angeordnet ist. Die Monomerflüssigkeit kann aber auch in einem Folienbeutel als separater Behälter enthalten sein oder sie kann in dem integrierten Behälter enthalten sein, der durch die Aufnahme beziehungsweise die Vakuummischvorrichtung selbst gebildet wird.

Der Begriff "Vakuummischvorrichtung" soll nicht in dahingehend falsch verstanden werden, dass ein Vakuum mit irgendetwas gemischt wird, sondern dass die Ausgangskomponenten des Knochenzements, also die Monomerflüssigkeit und das Zementpulver, unter Vakuum oder unter einem Druck kleiner als der Umgebungsdruck (Unterdruck) gemischt werden können.

Der Begriff Unterdruck bezieht sich vorliegend immer auf einen relativ zur umgebenden Atmosphäre bezogenen Druck, der also kleiner ist als der umgebende Atmosphärendruck.

Aufgrund der speziellen Anforderungen, wie die des geringen Volumens des Innenraums der Kartusche, sind aufwendigere Pumpensysteme nicht notwendig.

Bevorzugt kann vorgesehen sein, dass die Vakuumpumpe und die Druckpumpe in die Vakuummischvorrichtung integriert sind. Bevorzugt kann ferner vorgesehen sein, dass der Druck in dem Innenraum der zumindest einen Kartusche durch den Pumpvorgang um wenigstens 50% reduzierbar ist, bevorzugt um wenigstens 90% reduzierbar ist.

Bevorzugt kann erfindungsgemäß auch vorgesehen sein, dass das Zementpulver in dem Innenraum der Kartusche enthalten ist. Das Zementpulver muss dann nicht in den Innenraum der Kartusche eingefüllt werden.

Durch die Kopplung der Öffnungseinrichtung mit dem Bedienelement ist der Behälter in der Aufnahme oder ist der integrierte Behälter durch manuelles Bedienen des Bedienelements mit der Öffnungseinrichtung zu öffnen.

Evakuierbar bedeutet im Zusammenhang mit der vorliegenden Erfindung, dass ein Gas entfernbar ist, also beispielsweise ein Gas aus dem Innenraum der Kartusche abgesaugt werden kann, so dass anschließend im Innenraum der Kartusche ein Unterdruck verbleibt. Dieser Unterdruck kann zusätzlich zu dem durch die Druckpumpe aufgebrachten Druck zum Einsaugen der Monomerflüssigkeit genutzt werden.

Die Vakuummischvorrichtung umfasst entweder eine Aufnahme, in die ein separater Behälter, wie beispielsweise eine Glasampulle oder ein Folienbeutel, die oder der die Monomerflüssigkeit enthält, eingesetzt werden kann, oder einen integrierten Behälter, der als integraler Teil der Vakuummischvorrichtung ausgebildet ist und in dem die Monomerflüssigkeit bereits enthalten ist.

Es kann auch vorgesehen sein, dass mehr als eine Kartusche mit jeweils einem Innenraum vorgesehen ist, wobei dann in jedem Innenraum eine Mischeinrichtung vorgesehen ist und jeder Innenraum über eine Verbindungsleitung mit dem Vakuumpumpraum der Vakuumpumpe, dem Druckpumpraum der Druckpumpe oder mit jeweils einem Vakuumpumpraum einer Mehrzahl separater Vakuumpumpen und jeweils einem Druckpumpraum einer Mehrzahl separater Druckpumpen verbunden ist.

Bevorzugt ist die Aufnahme auch zur Fixierung einer Glasampulle beziehungsweise der Glasampulle in der Aufnahme geeignet und vorgesehen. Die Glasampulle muss hierzu selbstverständlich passend geformt sein. Beispielsweise kann die Glasampulle in Presspassung in die Aufnahme eingesteckt werden.

Es kann erfindungsgemäß vorgesehen sein, dass die Aufnahme auf einer Seite mit einem Deckel verschlossen ist. Dabei kann bevorzugt in dem Deckel zumindest eine gasdurchlässige Öffnung vorgesehen sein, durch die Gas in die Aufnahme einströmen beziehungsweise nachströmen kann, wenn die Monomerflüssigkeit aus der Aufnahme heraus fließt. Hierdurch soll vermieden werden, dass sich in der Aufnahme ein Unterdruck bildet, der dem Fluss der Monomerflüssigkeit in den Pumpraum der Druckpumpe entgegenwirkt.

Bevorzugt weist die Kartusche eine druckdichte Durchführung auf, durch die ein Stab, eine Kabel oder eine Mischwelle durchgeführt ist, mit dem die Mischeinrichtung von außerhalb der Kartusche zu bewegen ist. Dazu ist der Stab, das Kabel oder die Mischwelle bevorzugt drehbar und in Längsrichtung verschiebbar in der Durchführung gelagert. Mit der Mischeinrichtung kann der Inhalt der Kartusche gut durchmischt werden.

Bevorzugte Ausführungsformen können sich dadurch auszeichnen, dass die Vakuummischvorrichtung weniger als 10 kg Gesamtgewicht hat, besonders bevorzugt weniger als 2 kg Gesamtgewicht hat, besonders bevorzugt weniger als 1 kg.

Diese geringen Gewichte sind mit dem erfindungsgemäßen Aufbau der Vakuummischvorrichtung mit manuellem bedienbarem Bedienelement und der Vakuumpumpe möglich. Das geringe Gewicht hat den Vorteil, dass die Vakuummischvorrichtung mitgenommen werden kann, transportabel ist und ohne Anschluss an Versorgungsleitungen und ohne große Vorbereitungen einsetzbar ist.

Es kann erfindungsgemäß vorgesehen sein, dass ein Sieb und/oder ein Filter das, der oder die unterhalb der Aufnahme, des separaten Behälters oder unterhalb des integrierten Behälters angeordnet ist oder sind, so dass der Inhalt des geöffneten integrierten Behälters oder separaten Behälters durch das Sieb und/oder den Filter fließt.

Damit können Glassplitter, Folienschnipsel oder andere Rückstände des Verschlusses beziehungsweise des separaten oder integrierten Behälters, die beim Öffnen des separaten oder integrierten Behälters mit der Öffnungseinrichtung entstanden sind, zurückgehalten werden. Damit wird ein Verstopfen der Fluidverbindung zum Innenraum der Kartusche und ein Blockieren der Druckpumpe sowie eine Verunreinigung des herzustellenden Knochenzements verhindert. Erfindungsgemäße Vakuummischvorrichtungen zeichnen sich dadurch aus, dass sie ohne einen elektrischen Antrieb auskommen. Es kann also erfindungsgemäß vorgesehen sein, dass die Vakuummischvorrichtung keinen elektrischen Antrieb aufweist oder zumindest die Vakuumpumpe, die Druckpumpe, die Öffnungseinrichtung und die Mischeinrichtung nicht mit einem elektrischen Antrieb angetrieben werden. Stattdessen erfolgt der Antrieb dieser Bauteile erfindungsgemäß über das manuell bedienbare Bedienelement. Ebenso können erfindungsgemäße Vakuummischvorrichtungen ohne Elektronik beziehungsweise elektronische Bauteile aufgebaut werden. Eine erfindungsgemäße Vakuummischvorrichtung kann sich also auch dadurch auszeichnen, dass darin keine Elektronik verbaut ist oder dass zumindest zum Antrieb der Vakuumpumpe, der Druckpumpe, der Öffnungseinrichtung und der Mischeinrichtung keine Elektronik oder keine elektronischen Bauteile angewendet werden. Elektromotoren oder Kompressoren werden also nicht zum Aufbau erfindungsgemäßer Vakuummischvorrichtungen benötigt.

Des Weiteren kann erfindungsgemäß vorgesehen sein, dass die Vakuummischvorrichtung keinen Energiespeicher aufweist, insbesondere keinen elektrischen Energiespeicher, wie beispielsweise eine Batterie oder einen Akkumulator, und keinen Druckgasspeicher, wie beispielsweise eine CO₂-Druckgaspatrone, oder dass zumindest kein Energiespeicher, vorzugsweise kein elektrischer Energiespeicher oder Druckgasspeicher, zum Antrieb der Vakuumpumpe, der Öffnungseinrichtung und der Mischeinrichtung verwendet werden. Bevorzugt weist die Vakuummischvorrichtung aber auch keine elastischen Energiespeicher, wie beispielsweise gespannte Federn, auf.

Bei erfindungsgemäßen Vakuummischvorrichtungen kann vorgesehen sein, dass das Bedienelement mit dem Vakuumkolben und mit dem Pumpkolben derart verbunden oder verbindbar ist, dass der Vakuumkolben in der Vakuumpumpe und der Pumpkolben in der Druckpumpe durch Bedienen des Bedienelements manuell bewegbar ist.

Hiermit wird erreicht, dass der Vakuumkolben und der Pumpkolben, insbesondere unmittelbar, mit dem Bedienelement bewegt werden können. Dabei kann vorgesehen sein, dass sich der Vakuumkolben und der Pumpkolben erst nach einem ersten Bedienen des Bedienelements mit dem Bedienelement in der Art verbinden, dass der Vakuumkolben durch eine weitere Bedienung des Bedienelements in der Vakuumpumpe bewegt wird und dass der Pumpkolben durch eine weitere Bedienung des Bedienelements in der Druckpumpe bewegt wird. Für die Ausführung des Bedienelements ist dabei ein Hebel besonders gut geeignet, der um eine Achse hin und her gezogen beziehungsweise gedrückt beziehungsweise geschwenkt werden kann, so dass nach einem ersten Bewegen des Hebels der separate Behälter in der Aufnahme geöffnet wird oder der integrierte Behälter geöffnet wird und sich der Vakuumkolben, der Pumpkolben und/oder die Mischeinrichtung dabei mit dem Bedienelement derart verbindet oder verbinden, dass bei einer umgekehrten Bewegung des Hebels der Vakuumkolben in der Vakuumpumpe, der Pumpkolben in der Druckpumpe und/oder die Mischeinrichtung in dem Innenraum der Kartusche bewegt wird oder werden. Zudem sind mit einem Hebel problemlos große Kräfte manuell in die Vakuummischvorrichtung zu übertragen.

Des Weiteren kann vorgesehen sein, dass die Aufnahme zumindest bereichsweise geschlossene Seitenwände zur Aufnahme einer Glasampulle als separaten Behälter aufweist, wobei die Aufnahme zumindest eine verformbare geschlossene Seitenwand aufweist und gegenüber der verformbaren Seitenwand ein Stützelement vorgesehen ist, wobei die Öffnungseinrichtung über das Bedienelement gegen die verformbare Seitenwand der Aufnahme zu pressen ist, so dass sich die verformbare Seitenwand derart verformt, dass eine in der Aufnahme angeordnete passende Glasampulle mit der Öffnungseinrichtung aufzubrechen ist.

Durch diese Maßnahme kann die Aufnahme weitgehend nach außen geschlossen werden. Zudem kann hierdurch sichergestellt werden, dass eine Glasampulle auch innerhalb der geschlossenen Aufnahme geöffnet werden kann, ohne dass ohne weiteres Monomerflüssigkeit aus der Vakuummischvorrichtung austreten kann, wodurch die Gefahr einer Kontamination der Umgebung der Vakuummischvorrichtung mit dem Inhalt der Kartusche, insbesondere mit der Monomerflüssigkeit, ausgeschlossen werden kann oder die Gefahr hierfür zumindest stark reduziert wird.

Mit einer Weiterbildung der vorliegenden Erfindung wird vorgeschlagen, dass die Öffnungseinrichtung einen ersten Hebel aufweist, der um eine erste Achse drehbar gegen die Aufnahme oder den integrierten Behälter gelagert ist, wobei ein freies Ende des ersten Hebels gegen eine verformbare Seitenwand der Aufnahme oder den integrierten Behälter drückbar ist, wobei das Bedienelement durch einen zweiten Hebel gebildet ist, der um eine zweite Achse drehbar gegen die Aufnahme oder den integrierten Behälter gelagert ist, wobei die zweite Achse den zweiten Hebel in einen kurzen Hebelarm und einen langen Hebelarm teilt, wobei ein Ende des kurzen Hebelarms durch manuelles Bedienen des langen Hebelarms gegen den ersten Hebel zu drücken ist, so dass das freie Ende des ersten Hebels gegen die verformbare Seitenwand drückt und diese derart verformt, dass eine in der Aufnahme befindlicher separater Behälter zu öffnen ist, oder das freie Ende des ersten Hebels gegen den integrierten Behälter drückt, so dass der integrierte Behälter sich zu einer Fluidverbindung hin öffnet.

Dabei kann vorgesehen sein, dass der separate Behälter eine zur Aufnahme passende Glasampulle ist, die durch den Druck des freien Endes des ersten Hebels aufzubrechen ist oder ein in der Aufnahme angeordneter Folienbeutel ist, der durch den Druck des freien Endes des ersten Hebels aufzustechen, aufzuschlitzen oder aufzureißen ist.

Ferner kann bei solchen Ausführungen vorgesehen sein, dass am freien Ende des ersten Hebels auf der zur Aufnahme zugewandten Seite eine Kante angeordnet ist. Das Längenverhältnis des langen Hebelarms zum kurzen Hebelarm beträgt bevorzugt mindestens 5 zu 1. Des Weiteren kann vorgesehen sein, dass der zweite Hebel in der gleichen Ebene zu drehen ist, wie der erste Hebel, wobei die Bewegung des zweiten Hebels in die Bewegung des ersten Hebels eingreift. Bevorzugt kann auch vorgesehen sein, dass die zweite Achse des zweiten Hebels oberhalb der ersten Achse des ersten Hebels angeordnet ist, wobei vorzugsweise die erste Achse des ersten Hebels und die zweite Achse des zweiten Hebels parallel zueinander angeordnet sind.

Mit derartigen Vakuummischvorrichtungen mit Glasampulle gelingt es, eine Glasampulle großflächig innerhalb der Vorrichtung beziehungsweise der Zementiervorrichtung aufzubrechen, so dass die Monomerflüssigkeit aus der Glasampulle in kurzer Zeit ausströmt und zum Mischen mit dem medizinischen Knochenzementpulver bereitsteht. Mit Hilfe der beiden Hebel, die in Wechselwirkung miteinander stehen, ist es möglich, den Druck auf die Glasampulle in Richtung des Sitzes der Glasampulle in der Aufnahme zu richten, so dass ein Ausweichen der Glasampulle aus der Aufnahme heraus ausgeschlossen werden kann. Gleichzeitig kann ein sehr genau definierter lokaler Druck auf die Glasampulle ausgeübt werden, mit dem die Glasampulle in der Vakuummischvorrichtung aufgebrochen werden kann. Mit Hilfe der verformbaren Seitenwand kann dafür gesorgt werden, dass die Kraft durch diese Seitenwand ins Innere der Aufnahme auf die Glasampulle übertragen wird, wobei die Aufnahme dabei geschlossen bleibt. Ein Austreten der Monomerflüssigkeit aus der Aufnahme kann somit ausgeschlossen werden. Mit Hilfe des Siebs und/oder des Filters können Glassplitter, die beim Öffnen der Glasampulle entstehen können, zurückgehalten werden. Die Monomerflüssigkeit ist dann zum Mischen mit dem Knochenzementpulver nutzbar.

Der besondere Vorteil der erfindungsgemäßen Vorrichtung besteht auch darin, dass beliebige Glasampullen, unabhängig von der Ampullenlänge und der Geometrie des Ampullenkopfs, sicher geöffnet werden können, wenn der Ampullendurchmesser gleich oder etwas größer ist als der Innendurchmesser des Ampullenhalters beziehungsweise der Aufnahme. Weiterhin ist es ein besonderer Vorteil, dass beim Brechen der Ampullenwand im Bereich des Ampullenbodens die in der Glasampulle enthaltene Flüssigkeit unabhängig von der Oberflächenspannung sofort komplett ausfließt. Dagegen fließt bei konventionellen Ampullenbrechern die Flüssigkeit nach Abtrennen des Ampullenkopfs durch den relativ engen Querschnitt des Ampullenhalses deutlich langsamer aus. Dabei werden halbwegs große Auslaufgeschwindigkeiten nur erzielt, wenn entweder die Glasampulle im Winkel von ca. 45° mit dem Ampullenhals nach unten gehalten wird oder wenn der Querschnitt des Ampullenhalses so groß ist, dass die Oberflächenspannung der Flüssigkeit den Meniskus der Flüssigkeit nicht im Ampullenhals halten kann.

Bevorzugt ist die Aufnahme ein Hohlzylinder. Ebenfalls bevorzugt besteht die Aufnahme aus einem Elastomer oder umfasst einen Einsatz aus einem Elastomer, wie beispielsweise ein Ethylen-Propylen-Dien-Kautschuk (EPDM).

Ferner kann dabei vorgesehen sein, dass in der Aufnahme ein Absatz zur Auflage der Glasampulle angeordnet ist, wobei der Absatz kleiner als die Hälfte der Fläche des Ampullenbodens oder des Ampullenquerschnitts ist. Dabei kann wiederum bevorzugt vorgesehen sein, dass der Absatz derart in der Aufnahme angeordnet ist, dass der Abstand zwischen dem Absatz und einem darunter angeordneten Sieb und/oder Filter gleich oder größer ist als der Außendurchmesser der einzusetzenden Glasampulle.

Mit einer besonders einfach und kostengünstig zu realisierenden Vakuummischvorrichtung wird vorgeschlagen, dass das Bedienelement manuell beweglich ist, vorzugsweise ein um eine Achse schwenkbarer Hebel ist, wobei das Bedienelement derart mit der Öffnungseinrichtung, der Vakuumpumpe, der Druckpumpe und der Mischeinrichtung in Wirkverbindung steht oder in Wirkverbindung zu bringen ist, dass bei einem ersten Bedienen des Bedienelements ein separater Behälter in der Aufnahme oder der integrierte Behälter zu öffnen ist und bei einem weiteren Bedienen des Bedienelements der Vakuumkolben in der Vakuumpumpe anzutreiben ist, der Pumpkolben in der Druckpumpe anzutreiben ist und die Mischeinrichtung im Innenraum anzutreiben ist.

Hiermit kann eine manuell aufzubringende Kraft, die auf das Bedienelement, insbesondere den Hebel, einwirkt, dazu verwendet werden, zunächst den separaten oder integrierten Behälter zu öffnen und anschließend die Vakuumpumpe, die Druckpumpe und die Mischeinrichtung mit demselben Bedienelement anzutreiben beziehungsweise zu bewegen. Hebel als Bedienelemente sind für die Übertragung manueller Kraft in die Vakuummischvorrichtung besonders gut geeignet. Auch weil über die Länge des Hebelarms eine Verstärkung der nutzbaren Kraft möglich ist.

Dabei kann vorgesehen sein, dass der Vakuumkolben der Vakuumpumpe, der Pumpkolben der Druckpumpe und/oder die Mischeinrichtung über ein flexibles Kabel und/oder eine Stange anzutreiben sind, wobei an dem flexiblen Kabel und/oder der Stange ein Rastmittel vorgesehen ist, das nach erstmaligem Bedienen des Bedienelements in ein Gegenrastmittel am Bedienelement oder in ein mit dem Bedienelement verbundenes Gegenrastmittel greift, so dass bei einem der Rastung nachfolgenden Bedienen des Bedienelements der Vakuumkolben der Vakuumpumpe, der Pumpkolben der Druckpumpe und/oder die Mischeinrichtung über das Kabel und/oder die Stange mit dem Bedienelement anzutreiben sind.

Hiermit kann eine Übertragung der Kraft von dem Bedienelement auf den Vakuumkolben, den Pumpkolben der Druckpumpe und/oder die Mischeinrichtung erfolgen. Die flexiblen Kabel sind besonders gut zur Übertragung der Kraft geeignet, da sich damit ohne aufwendige Mechanik die Richtung der Kraft umlenken lässt. Vorzugsweise ist das flexible Kabel ausreichend steif beziehungsweise starr, so dass sich der Vakuumkolben, der Pumpkolben und/oder vor allem die Mischeinrichtung in beide Richtungen hin und her bewegen lässt. Hierzu kann das Kabel geführt und oder gestützt werden. Beispielsweise kann das Kabel hierzu in einem Kanal geführt sein oder es kann an geeigneten Stellen durch ein Gehäuse und durch Streben und/oder Umlenkrollen im Gehäuse gestützt beziehungsweise umgelenkt werden. Durch die Anwendung des Rastmittels und des Gegenrastmittels kann sichergestellt werden, dass zunächst der separate Behälter in der Aufnahme oder der integrierte Behälter geöffnet wird und die Monomerflüssigkeit vollständig ausläuft, bevor die Druck-Vakuumpumpe beziehungsweise der Vakuumkolben der Vakuumpumpe, der Pumpkolben der Druckpumpe und/oder die Mischeinrichtung bedient wird. Damit kann beispielsweise verhindert werden, dass die Vakuumpumpe und die Druckpumpe schon betrieben wird, bevor die Monomerflüssigkeit zur Verfügung steht.

Es wird ferner vorgeschlagen, dass die Mischeinrichtung durch Bedienen des Bedienelements in dem Innenraum axial in Längsrichtung beweglich ist.

Hiermit wird insbesondere für einen zylindrischen Innenraum eine sehr weitreichende Durchmischung des Innenraums erreicht. Gleichzeitig kann die Mischeinrichtung kompakt aufgebaut werden, da sie sich nicht entlang der gesamten Länge des Innenraums erstrecken muss.

Bevorzugt kann auch vorgesehen sein, dass die Mischeinrichtung durch Bedienen des Bedienelements in dem Innenraum um die Längsachse des Innenraums drehbar ist, wobei hierzu vorzugsweise sich ein mit der Mischeinrichtung verbundener Zylinder mit einem Außengewinde in einer feststehenden Hülse mit einem passenden Innengewinde bewegt, so dass bei einer Bewegung des Zylinders entlang der Längsrichtung innerhalb der Hülse eine Drehung des Zylinders erzwungen wird, wobei sich die Drehung des Zylinders auf die Mischeinrichtung im Innenraum der Kartusche überträgt.

Hierdurch wird eine noch stärkere Durchmischung des Inhalts des Innenraums erreicht. Auch an der Wand des Innenraums anliegender Knochenzement kann dadurch effektiv durchmischt werden. Der Knochenzement wird so schneller und effektiver durchmischt.

Mit einer Weiterbildung der vorliegenden Erfindung kann des Weiteren vorgesehen sein, dass der Vakuumpumpraum der Vakuumpumpe gasdicht ist und im Inneren der Vakuumpumpe angeordnet ist, wobei der Vakuumkolben über das Bedienelement manuell in zumindest einer Richtung antreibbar ist, so dass durch die Bewegung des Vakuumkolbens der Vakuumpumpraum zu vergrößern ist und mit dem dadurch im Vakuumpumpraum entstehenden Unterdruck der Innenraum der zumindest einen Kartusche durch die Verbindungsleitung evakuierbar ist.

Hiermit wird eine besonders gute Pumpwirkung der Vakuumpumpe erreicht und damit das in dem Innenraum der Kartusche erzeugbare Vakuum verbessert. Besonders bevorzugt lässt sich der Vakuumkolben mit dem Bedienelement manuell wiederholt in zwei Richtungen entlang der Achse des Vakuumpumpraums bewegen, so dass ein wiederholtes Pumpen zum Evakuieren des Innenraums der Kartusche möglich ist.

Dabei kann vorgesehen sein, dass die Volumenvergrößerung des Vakuumpumpraums mindestens so groß ist wie das freie Volumen des Innenraums der Kartusche, bevorzugt die Volumenvergrößerung des Vakuumpumpraums mindestens so groß ist wie die Summe des Volumens des Innenraums der Kartusche, in dem das Zementpulver des PMMA-Knochenzements enthalten ist, und des Volumens der Verbindungsleitung und des Volumens einer Fluidverbindung zwischen dem Innenraum der Kartusche und der Aufnahme für den separaten Behälter oder dem integrierten Behälter und des Volumens einer mit dem Zementpulver des PMMA-Knochenzements in der Kartusche zu mischenden Monomerflüssigkeit.

Hierdurch wird sichergestellt, dass die Vakuumpumpe den Innenraum der Kartusche bereits mit einem Hub oder mit wenigen Hüben des Vakuumkolbens evakuieren kann. Dabei ist das Volumen des Vakuumpumpraums vor dem Pumpvorgang idealerweise möglichst klein. Es kann also bevorzugt vorgesehen sein, dass das Volumen des Vakuumpumpraums nach dem Pumpvorgang zumindest 10 mal größer ist als das Volumen des Vakuumpumpraums vor dem Pumpvorgang, besonders bevorzugt zumindest 20 mal größer ist als das Volumen des Vakuumpumpraums vor dem Pumpvorgang. Bevorzugt liegt der Vakuumkolben nicht derart dicht an dem Innenraum der Vakuumpumpe, insbesondere der Deckfläche eines Hohlzylinders mit dem Anschluss für die Verbindungsleitung, flächenbündig an, dass sich der Vakuumkolben nicht an dieser Fläche festsaugt und zum Starten der Bewegung nur schwer bewegt werden kann. Hierzu können Abstandhalter an der Deckfläche im Vakuumpumpraum und/oder an dem Vakuumkolben im Vakuumpumpraum vorgesehen sein.

Ferner kann vorgesehen sein, dass der Druckpumpraum der Druckpumpe flüssigkeitsdicht ist und im Inneren der Druckpumpe angeordnet ist, wobei der Pumpkolben über das Bedienelement manuell in zumindest einer Richtung antreibbar ist, so dass durch die Bewegung des Pumpkolbens der Druckpumpraum zu verkleinern ist und mit dem dadurch im Druckpumpraum entstehenden Druck die Monomerflüssigkeit aus dem Druckpumpraum durch die Fluidverbindung in den Innenraum der zumindest einen Kartusche zu drücken ist.

Hiermit wird eine besonders gute Pumpwirkung der Druckpumpe erreicht. Die Monomerflüssigkeit kann so besonders effektiv und durch Bedienen des Bedienelements aus dem Druckpumpraum in den Innenraum der Kartusche getrieben werden.

Um die Vakuumpumpe mit mehreren Bedienungen des Bedienelements bedienen zu können, beziehungsweise einen mehrfachen Hub des Vakuumkolbens der Vakuumpumpe nutzen zu können, kann vorgesehen sein, dass in der Verbindung vom Vakuumpumpraum zu der Verbindungsleitung oder in der Verbindungsleitung ein erstes Einwegventil angeordnet ist, das offen ist oder öffnet, wenn im Vakuumpumpraum ein geringerer Druck herrscht als im Innenraum der Kartusche, und geschlossen ist oder schließt, wenn im Vakuumpumpraum ein gleicher oder ein höherer Druck herrscht als im Innenraum der Kartusche, und bevorzugt ein zweites Einwegventil den Vakuumpumpraum mit der Umgebung der Vakuumpumpe verbindet, das offen ist oder öffnet, wenn im Vakuumpumpraum ein höherer Druck herrscht als in der Umgebung der Vakuumpumpe, und geschlossen ist oder schließt, wenn im Vakuumpumpraum ein gleicher oder ein geringerer Druck herrscht als in der Umgebung der Vakuumpumpe, wobei die Einwegventile bevorzugt Rückschlagventile sind.

Mit dem ersten Einwegventil wird erreicht, dass eine Strömung von dem Innenraum der Kartusche in den Vakuumpumpraum möglich ist, umgekehrte Strömungen aber weitgehend verhindert werden. Durch das zweite Einwegventil wird erreicht, dass ein Überdruck im Vakuumpumpraum über das zweite Einwegventil abgeblasen werden kann.

Hiermit kann mit einem wiederholten Pumpen durch mehrfache Bewegung des Vakuumkolbens aufgrund eines mehrfachen Bedienens des Bedienelements ein besonders gutes Vakuum beziehungsweise ein besonders guter Unterdruck im Innenraum der Kartusche erzeugt werden, der sich auch während dem Mischvorgang aufrechterhalten beziehungsweise noch verbessern lässt.

Des Weiteren kann vorgesehen sein, dass der der Vakuumkolben und der Pumpkolben in einem Hohlzylinder axial beweglich gelagert sind, wobei der Hohlzylinder auf einer ersten Seite geschlossen ist oder bis auf eine Durchführung für eine mit dem Bedienelement und dem Vakuumkolben und Pumpkolben verbundene Stange oder verbundenen Kabel geschlossen ist, insbesondere durch einen Verschluss geschlossen ist, wobei vorzugsweise ein Vakuumpumpraum in dem Hohlzylinder zwischen dem Vakuumkolben und der ersten geschlossenen Seite gebildet ist und ein Druckpumpraum in dem Hohlzylinder zwischen dem Pumpkolben und einem Verschluss gebildet ist, wobei der Verschluss den Hohlzylinder auf der der ersten Seite gegenüberliegenden Seite des Hohlzylinders angeordnet ist.

Bevorzugt haben der Vakuumpumpraum und der Vakuumkolben sowie der Druckpumpraum und der Druckkolben eine zylindrische Geometrie. Durch diese Maßnahmen und auch durch die zylindrische Geometrie wird eine besonders einfach und kostengünstig zu fertigende kombinierte Druck-Vakuumpumpe vorgeschlagen, die leicht zu bedienen ist und die besonders unanfällig für Fehlfunktionen ist.

Es wird auch vorgeschlagen, dass der Vakuumkolben und oder der Pumpkolben über eine Stange und/oder ein Kabel mit dem Bedienelement verbunden oder verbindbar ist oder sind und vorzugsweise der Vakuumkolben durch Bedienen des Bedienelements in der Vakuumpumpe zu bewegen ist und/oder der Pumpkolben durch Bedienen des Bedienelements in der Druckpumpe zu bewegen ist.

Hierdurch wird eine besonders einfache Vakuummischvorrichtung bereitgestellt, bei der keine große Gefahr für mögliche Störungen besteht. Die direkte Verbindung des Bedienelements mit dem Vakuumkolben über das Kabel und/oder die Stange kann mit einem einteiligen Spritzgussteil aus Kunststoff realisiert werden. Alternativ kann auch eine Übersetzung beziehungsweise ein Getriebe vorgesehen sein, mit dem die Kraft, die auf das Bedienelement ausgeübt wird, zum Vakuumkolben übersetzt wird, um eine kräftigere Bewegung des Vakuumkolbens zu ermöglichen.

Bei einer Weiterentwicklung der Vakuummischvorrichtung kann vorgesehen sein, dass in dem Innenraum der Kartusche ein beweglicher Austragskolben zum Austragen des gemischten Knochenzements aus der Kartusche angeordnet ist, wobei der Austragskolben vorzugsweise lösbar arretiert oder arretierbar ist, um eine Bewegung des Austragskolbens unter Einwirkung des Unterdrucks zu verhindern.

Mit dem Austragskolben wird die Bedienung der Vakuummischvorrichtung vereinfacht.

Dabei kann vorgesehen sein, dass der Austragskolben einen Durchgang mit einer gasdurchlässige Porenscheibe aufweist, die für Zementpulver undurchlässig ist, wobei der Durchgang mit der Porenscheibe den Innenraum der Kartusche mit der Verbindungsleitung und/oder der Umgebung gasdurchlässig verbindet, wobei der Durchgang gasdicht verschließbar ist, vorzugsweise mit einem Dichtungskolben des Austragskolbens gasdicht verschließbar ist.

Mit der Porenscheibe kann sichergestellt werden, dass der Innenraum der Kartusche mit dem Zementpulver darin mit Hilfe eines Gases wie Ethylenoxid sterilisiert werden kann, ohne dass die Gefahr besteht, dass das Zementpulver aus dem Innenraum der Kartusche nach außen in die Umgebung gelangt.

Bevorzugt kann auch vorgesehen sein, dass die Kartusche eine mit Zementpulver gefüllte Zementkartusche ist und in der Aufnahme ein separater Behälter enthaltend eine Monomerflüssigkeit angeordnet ist oder in dem integrierten Behälter eine Monomerflüssigkeit enthalten ist.

Hiermit wird erreicht, dass die Monomerflüssigkeit aus dem separaten oder integrierten Behälter mit der gleichen Pumpbewegung beziehungsweise dem gleichen Pumpvorgang in den Innenraum der Kartusche überführt werden kann, mit dem der Innenraum der Kartusche evakuiert wird. Das Vakuum beziehungsweise der Unterdruck, der mit der Vakuumpumpe manuell erzeugt wird, wird dabei gleichzeitig zum Einsaugen der Monomerflüssigkeit in die Kartusche genutzt.

Es kann vorgesehen sein, dass die Aufnahme oder der integrierte Behälter durch ein zu öffnendes Trennelement flüssigkeitsundurchlässig mit dem Innenraum der Zementkartusche verbunden ist und/oder der Innenraum der Zementkartusche mit der Vakuumpumpe gasdurchlässig verbunden ist oder verbindbar ist.

Ferner kann vorgesehen sein, dass die Kartusche, die Vakuumpumpe, die Druckpumpe und alle Leitungen sowie die Aufnahme oder der integrierte Behälter mit einem gemeinsamen Fußteil und/oder einem Gehäuse fest und/oder lösbar verbunden sind, wobei vorzugsweise die Vakuumpumpe, die Druckpumpe und alle Leitungen sowie die Aufnahme oder der separate Behälter fest mit dem Fußteil und/oder dem Gehäuse verbunden sind und die Kartusche lösbar mit dem Fußteil und/oder dem Gehäuse verbunden ist.

Eine derartige Vakuummischvorrichtung kann einfach aufgestellt werden und ist leicht bedienbar. Die Anwendung der Vakuummischvorrichtung wird dadurch vereinfacht. Am Ort der Anwendung muss dann lediglich eine ebene Unterlage zum Aufstellen der Vakuummischvorrichtung vorhanden sein, was in den meisten OP-Bereichen kein Problem darstellt.

Gemäß einer Ausführungsform kann vorgesehen sein, dass der separate Behälter enthaltend die Monomerflüssigkeit ein Folienbeutel ist, der in der Aufnahme mit der Öffnungseinrichtung aufschneidbar oder aufreißbar ist, oder eine Glasampulle ist, die in der Aufnahme mit der Öffnungseinrichtung aufbrechbar ist.

Hierdurch können handelsübliche Verpackungen der Monomerflüssigkeit eingesetzt werden, ohne dass diese außerhalb der Vakuummischvorrichtung geöffnet werden müssen.

Es wird vorgeschlagen, dass die Aufnahme oder der integrierte Behälter über einen Trichter mit dem Druckpumpraum der Druckpumpe verbunden ist. Die Einmündung des Trichters in den Druckpumpraum soll dabei möglichst großflächig sein aber einen parallel zur Bewegung des Pumpkolbens schmalen Durchmesser aufweisen, damit einerseits die Monomerflüssigkeit möglichst ungehindert in den Pumpraum fließen kann und andererseits die Einmündung des Trichters in den Pumpraum durch den Pumpkolben möglichst leicht und durch eine kurze Bewegung abgedeckt werden kann, damit die Monomerflüssigkeit nicht aus dem Pumpraum in die Aufnahme beziehungsweise den integrierten Behälter zurück gedrückt wird.

Es kann vorgesehen sein, dass die Aufnahme oder der integrierte Behälter über eine Fluidverbindung mit dem Innenraum der Kartusche verbunden oder verbindbar ist. Bevorzugt ist die Einmündung der Fluidverbindung in den Innenraum der Kartusche auf der gegenüberliegenden Seite der Einmündung der Verbindungsleitung zwischen dem Innenraum und dem Vakuumpumpraum angeordnet.

Hiermit wird erreicht, dass die Monomerflüssigkeit über eine eigene Leitung (die Fluidverbindung) in den Innenraum der Kartusche geleitet werden kann.

Mit einer Weiterbildung der vorliegenden Erfindung wird ferner vorgeschlagen, dass durch die Bewegung des Vakuumkolbens in der Vakuumpumpe im Vakuumpumpraum ein Unterdruck zu erzeugen ist, wobei mit dem Unterdruck ein Gas durch die Verbindungsleitung aus dem Innenraum der zumindest einen Kartusche evakuierbar ist.

Hiermit wird ein besonders einfacher und unanfälliger Aufbau bereitgestellt.

Erfindungsgemäß kann auch vorgesehen sein, dass die Vakuumpumpe und die Druckpumpe als kombinierte Druck-Vakuumpumpe aufgebaut ist, die Druck-Vakuumpumpe aufweisend einen Hohlzylinder, mit einem gasdichten Verschluss an einem ersten Hohlzylinderende und einen flüssigkeitsdichten Verschluss an dem gegenüberliegenden zweiten Hohlzylinderende, wobei der Hohlzylinder an dem ersten und an dem zweiten Hohlzylinderende mit dem Innenraum der Kartusche verbunden oder verbindbar ist, den Vakuumkolben, der im Hohlzylinder gasdicht, axial beweglich angeordnet ist, und den Pumpkolben, der im Hohlzylinder flüssigkeitsdicht, axial beweglich angeordnet ist, wobei der Vakuumkolben und der Pumpkolben in der Druck-Vakuumpumpe mit dem manuell bedienbaren Bedienelement bewegbar sind, wobei bei einem Bewegen des Vakuumkolbens mit dem manuell bedienbaren Bedienelement der Vakuumkolben axial vom gasdichten Verschluss weg bewegbar ist und dadurch Gas aus dem Innenraum der Kartusche evakuiert, und bei einem Bewegen des Pumpkolbens mit dem manuell bedienbaren Bedienelement der Pumpkolben axial zum flüssigkeitsdichten Verschluss hin bewegbar ist und dadurch eine Monomerflüssigkeit aus dem Pumpraum in den Innenraum der Kartusche hinein drückbar ist, wobei das Bedienelement mit der Öffnungseinrichtung in Wirkverbindung steht und wobei das Bedienelement mit der Mischeinrichtung im Innenraum der Kartusche derart verbunden ist, dass bei einem Bedienen des Bedienelements die Mischeinrichtung im Innenraum der Kartusche bewegbar ist.

Dieser Aufbau ist besonders einfach und die wesentlichen Teile hierfür können aus Kunststoff durch Spritzgießen gefertigt werden.

Mit einer besonders bevorzugten Ausführung der vorliegenden Erfindung kann vorgesehen sein, dass die Vakuumpumpe, die Druckpumpe, die Öffnungseinrichtung und die Mischeinrichtung über die Bewegung des Bedienelements antreibbar sind, wobei bevorzugt die Bewegung des Bedienelements durch manuelle Krafteinwirkung erfolgt.

Hierdurch wird erreicht, dass die Vakuummischvorrichtung keinerlei Energiespeicher und keine elektrischen oder elektronischen Antriebe benötigt. Dies ist deswegen erstrebenswert, da die Vakuummischvorrichtung zum einmaligen Gebrauch bestimmt ist und auf diese Weise leichter zu recyceln ist. Zudem kann so erreicht werden, dass die Vakuummischvorrichtung grundsätzlich anwendbar ist und keine Anschlüsse, wie Kabel oder Druckgasschläuche benötigt, um eingesetzt werden zu können.

Mit einer Weiterbildung des Vakuummischsystems kann vorgesehen sein, dass die Vakuumpumpe und die Druckpumpe als kombinierte Druck-Vakuumpumpe ausgeführt sind, wobei vorzugsweise der Vakuumkolben und Pumpkolben einteilig oder miteinander über eine Verbindung, insbesondere über eine Stange oder ein Rohr, verbunden sind.

Hierdurch wird der Aufbau vereinfacht. Zudem können so der Pumpkolben und der Vakuumkolben leicht gemeinsam angetrieben werden, so dass keine separaten oder kombinierten Anschlüsse an das Bedienelement notwendig sind. Der Pumpkolben und der Vakuumkolben laufen so in jedem Fall synchron. Ferner kann der Gesamtaufbau so kompakter und leichter gestaltet werden.

Dabei kann vorgesehen sein, dass der Abstand zwischen den voneinander abgewandten Seiten des Vakuumkolbens und des Pumpkolbens zumindest genauso groß ist wie der maximale Hub des Vakuumkolbens und des Pumpkolbens.

Auf diese Weise kann verhindert werden, dass der Zulauf für die Monomerflüssigkeit aus der Aufnahme oder dem integrierten Behälter mit dem Vakuumpumpraum verbunden werden kann. Dieser Zulauf ist bevorzugt derart positioniert, dass er angrenzend oder in unmittelbarer Nähe des Pumpkolbens in seiner Ausgangsstellung in der Mantelfläche des Druckpumpraums angeordnet ist, so dass der Zulauf bereits am Anfang des ersten Hubs von dem Pumpkolben überfahren und damit geschlossen wird. Dementsprechend liegt der Zulauf dann in Reichweite des Vakuumkolbens, so dass durch die Beabstandung verhindert werden kann, dass Monomerflüssigkeit in den Vakuumpumpraum gelangen kann. Alternativ hierzu kann auch eine Verschlusseinrichtung vorgesehen sein, die den Zulauf bei der ersten Bewegung des Pumpkolbens dicht verschließt.

Mit einer besonders bevorzugten Ausführung der vorliegenden Erfindung kann vorgesehen sein, dass der Pumpkolben nur in eine Richtung zur Verkleinerung des Druckpumpenraums bewegbar ist und anschließend nicht wieder in die entgegengesetzte Richtung bewegbar ist, vorzugsweise von der Verbindung nur in eine Richtung zur Verkleinerung des Druckpumpenraums bewegbar ist.

Hierdurch kann sichergestellt werden, dass sich der Druckpumpenraum nicht wieder vergrößert, so dass durch weiteres Betreiben der Vakuumpumpe der Innenraum der Kartusche weiter evakuiert werden kann, ohne dass Luft aus dem Druckpumpenraum und über die offene Aufnahme oder den offenen internen Behälter nachströmen kann. Dazu schließt der Pumpkolben bevorzugt in seiner Endstellung die Fluidverbindung beziehungsweise die Einmündung zur Fluidverbindung druckdicht ab.

Die der vorliegenden Erfindung zugrundeliegenden Aufgaben werden auch gelöst durch ein Verfahren zur Vermischung von Polymethylmethacrylat-Knochenzement in einem Innenraum einer Kartusche einer Vakuummischvorrichtung, insbesondere einer solchen, oben beschriebenen Vakuummischvorrichtung, bei dem
ein Bedienelement bedient wird und dadurch ein integrierter Behälter der Vakuummischvorrichtung oder ein separater Behälter, der in einer Aufnahme der Vakuummischvorrichtung angeordnet ist, geöffnet wird, wobei anschließend eine in dem integrierten Behälter oder dem separaten Behälter enthaltene Monomerflüssigkeit als erste Komponente des Knochenzements in einen Pumpraum einer Druckpumpe fließt,
durch eine anschließende weitere Bedienung des Bedienelements eine Bewegung eines Vakuumkolbens einer Vakuumpumpe der Vakuummischvorrichtung angetrieben wird, wobei durch die Bewegung des Vakuumkolbens ein Unterdruck in einem Vakuumpumpraum der Vakuumpumpe erzeugt wird, wobei mit der derart angetriebenen Vakuumpumpe der Innenraum der Kartusche evakuiert wird,
durch die weitere Bedienung des Bedienelements eine Bewegung eines Pumpkolbens einer Druckpumpe der Vakuummischvorrichtung angetrieben wird, wobei durch die Bewegung des Pumpkolbens ein Druck auf die Monomerflüssigkeit im Pumpraum ausgeübt wird und die Monomerflüssigkeit aus dem Pumpraum in den Innenraum der Kartusche gedrückt wird, wobei sich in dem Innenraum der Kartusche bereits ein Knochenzementpulver als zweite Komponente des Knochenzements befindet, und
durch die Bedienung des Bedienelements eine Mischeinrichtung im Innenraum der Kartusche bewegt wird und durch die Bewegung der Mischeinrichtung ein Knochenzementteig im Innenraum der Kartusche aus dem Zementpulver und der Monomerflüssigkeit gemischt wird.

Dabei kann vorgesehen sein, dass das Volumen eines Vakuumpumpraums der Vakuumpumpe durch die manuelle Bewegung des Vakuumkolbens vergrößert wird und durch den dadurch entstehenden Unterdruck der Innenraum der Kartusche evakuiert wird.

Alternativ oder zusätzlich kann vorgesehen sein, dass das Volumen des Druckpumpraums der Druckpumpe durch die manuelle Bewegung des Pumpkolbens verkleinert wird und durch den dadurch entstehenden Druck die Monomerflüssigkeit aus dem Druckpumpraum in den Innenraum der Kartusche gedrückt wird.

Hiermit kann die Vakuumpumpe beziehungsweise die Druckpumpe auf einfache Weise realisiert werden.

Ferner kann erfindungsgemäß vorgesehen sein, dass ein Zementpulver in dem Innenraum der Kartusche enthalten ist und ein Gas aus dem Innenraum der Kartusche mit der Vakuumpumpe evakuiert wird, mit der Druckpumpe eine Monomerflüssigkeit in den Innenraum der Kartusche gedrückt wird und die Monomerflüssigkeit mit dem Zementpulver in dem evakuierten Innenraum der Kartusche durch eine Bewegung der Mischeinrichtung gemischt wird.

Durch die genannte Kombination und Wechselwirkung der Verfahrensschritte kann ein besonders einfacher und sicher zu realisierender Aufbau erreicht werden.

Des Weiteren kann vorgesehen sein, dass der Vakuumkolben der Vakuumpumpe mit dem Bedienelement bewegt wird, wodurch ein Unterdruck gegenüber der umgebenden Atmosphäre in der Vakuumpumpe erzeugt wird, dabei durch eine Verbindungsleitung Gas aus dem Innenraum der Kartusche in den Vakuumpumpraum der Vakuumpumpe gesaugt wird, und der Pumpkolben der Druckpumpe mit dem Bedienelement bewegt wird, wodurch ein Druck auf die Monomerflüssigkeit in dem Druckpumpraum ausgeübt wird, die Monomerflüssigkeit durch eine Fluidverbindung vom Druckpumpraum in den Innenraum der Kartusche gedrückt wird, anschließend durch Bedienen desselben Bedienelements die Vakuummischvorrichtung im Innenraum der Kartusche bewegt wird und dabei das Zementpulver mit der Monomerflüssigkeit vermischt wird, anschließend die Kartusche mit dem gemischten Zementteig entnommen wird und durch axiale Bewegung eines Austragskolbens der Zementteig aus der Kartusche heraus gepresst wird.

Hiermit wird das Verfahren dahingehend komplettiert, dass am Ende eine Zementkartusche mit einem unter Vakuum gemischten Knochenzementteig bereitgestellt wird, der unmittelbar angewendet werden kann.

Schließlich kann auch vorgesehen sein, dass das Zementpulver in der Kartusche angeordnet ist, die Monomerflüssigkeit in einer von der Kartusche separaten Aufnahme angeordnet ist, wobei die Monomerflüssigkeit in einem integrierten Behälter oder in einem separaten Behälter, vorzugsweise in einer Glasampulle in der Aufnahme, enthalten ist, der integrierte Behälter oder der separate Behälter durch Bedienen des Bedienelements und einer daraus resultierenden Bewegung der Öffnungseinrichtung geöffnet wird, bevor der Vakuumkolben und der Pumpkolben durch ein weiteres Bedienen des Bedienelements angetrieben werden, danach der Vakuumkolben und der Pumpkolben axial in einem Hohlzylinder bewegt werden, dabei durch die Verbindungsleitung Gas aus dem Innenraum der Kartusche in den durch den Hohlzylinder begrenzten Vakuumpumpraum gesaugt wird und die in dem durch den Hohlzylinder begrenzten Druckpumpraum befindliche Monomerflüssigkeit durch die Fluidverbindung in den Innenraum der Kartusche gedrückt wird.

Dadurch wird das Verfahren weiter komplettiert.

Erfindungsgemäße Verfahren können auch durch die bestimmungsgemäße Anwendung oder Verwendung von Bauteilen erfindungsgemäßer Vakuummischvorrichtungen gekennzeichnet sein.

Der Erfindung liegt die überraschende Erkenntnis zugrunde, dass es mit einem einzigen Bedienelement gelingt, sowohl die Vakuumpumpe, die Druckpumpe (beziehungsweise die kombinierte Druck-Vakuumpumpe) und die Mischeinrichtung zu betreiben beziehungsweise anzutreiben, als auch die Öffnungseinrichtung zu bedienen und anzutreiben. Dies hat den Vorteil, dass keine komplizierten Handlungsanweisungen an das bedienende Personal notwendig sind. Durch Bedienen des einzigen Bedienelements können alle Abläufe gesteuert und angetrieben werden. Die Vakuummischvorrichtung wird so maximal vereinfacht. Gleichzeitig sind keine Energiespeicher zum Antrieb notwendig und es bedarf keiner elektrischen beziehungsweise elektronischen Steuerung, um die Vakuumpumpe, die Druckpumpe, die Mischeinrichtung und die Öffnungseinrichtung anzutreiben und zu steuern.

Gleichzeitig gelingt es mit Hilfe der manuell anzutreibenden Vakuumpumpe und manuell anzutreibenden Druckpumpe, eine von inneren und äußeren Energiequellen und anderen Versorgungsleitungen unabhängige Vakuummischvorrichtung bereitzustellen. Die erfindungsgemäße Vakuummischvorrichtung kann kompakt, leicht und platzsparend aufgebaut werden. Die Vakuumpumpe, die Druckpumpe, die Öffnungseinrichtung und die gesamte Vakuummischvorrichtung können mit einfachsten Mitteln aufgebaut werden, so dass die gesamte Vakuummischvorrichtung als Einmalsystem verwendet werden kann. Die Druckpumpe wird dazu eingesetzt, um eine Monomerflüssigkeit in das Zementpulver zu überführen, während die Vakuumpumpe vornehmlich zum Evakuieren des Innenraums der Kartusche genutzt wird, so dass der Knochenzement unter Unterdruck beziehungsweise unter Vakuum gemischt werden kann. Zusätzlich unterstützt das Vakuum beziehungsweise der Unterdruck im Innenraum der Kartusche den Transport der Monomerflüssigkeit in den Innenraum der Kartusche, da die Monomerflüssigkeit durch die Fluidverbindung angesaugt wird. Die beiden Komponenten des PMMA-Knochenzements können dann im Vakuum beziehungsweise in dem Unterdruck gemischt werden.

In Zementiersystemen nach der vorliegenden Erfindung ist eine Vorrichtung zur Erzeugung eines Vakuums beziehungsweise zur Erzeugung eines Unterdrucks enthalten, die zur temporären Erzeugung eines Unterdrucks vor und während der Vermischung einer pulverförmigen Komponente mit einer flüssigen Monomerkomponente des Polymethylmethacrylat-Knochenzements geeignet ist.

Die der Erfindung zugrundeliegende Idee basiert auf der Erkenntnis, dass nur eine relativ geringe Energiemenge und damit ein geringer manueller Kraftaufwand notwendig ist, um den Behälter für die Monomerflüssigkeit zu öffnen, um die Monomerflüssigkeit in den Innenraum der Kartusche zu drücken, um das Vakuum beziehungsweise den Unterdruck in einer Kartusche zu erzeugen, der notwendig ist, um die Ausgangskomponenten eines Knochenzements unter dem Unterdruck oder dem Vakuum zu mischen, und um die Mischeinrichtung zum Durchmischen des Knochenzementteigs im Innenraum der Kartusche zu bewegen. Diese geringe Energiemenge kann ohne weiteres durch Bedienen eines Hebels als Bedienelement aufgebracht werden. Hierdurch ist die Vakuummischvorrichtung einfach in der Handhabung und leicht zu bedienen sowie unabhängig von inneren und äußeren Energiespeichern. Durch einen geeigneten Aufbau lässt sich auch die Reihenfolge der Abläufe steuern, nämlich dass zuerst der Monomerbehälter geöffnet wird und erst anschließend die Vakuumpumpe, die Druckpumpe und die Mischeinrichtung angetrieben werden.

Die Idee der Erfindung beruht auch darauf, dass durch manuelle Betätigung eines Bedienelements mit einem damit verbundenen Vakuumkolben in einem Hohlzylinder der Vakuumpumpe ein Unterdruck in dem Hohlzylinder der Vakuumpumpe erzeugt wird, wobei sich der Unterdruck über ein Leitungsmittel in die Kartusche ausbreitet, gleichzeitig durch manuelle Betätigung eines Bedienelements mit einem damit verbundenen Pumpkolben in einem Hohlzylinder der Druckpumpe oder in dem gleichen Hohlzylinder der als kombinierte Druck-Vakuumpumpe ausgeführten Druckpumpe ein Druck in dem Hohlzylinder der Druckpumpe auf die darin enthaltene Monomerflüssigkeit ausgeübt wird und die Monomerflüssigkeit in die Kartusche gedrückt und gesaugt wird, in dem sich Zementpulver befindet. Danach erfolgt eine manuelle Vermischung der Zementkomponenten mit Hilfe einer Mischeinrichtung, die über dasselbe Bedienelement zeitgleich anzutreiben ist.

Beispielsweise kann die Erfindung mit dem folgenden Verfahren umgesetzt werden, bei dem durch Aktivierung mit einem manuell zu bedienenden Hebel oder allgemeiner einem manuell zu bedienenden Bedienelement die folgenden Funktionen in der Mischvorrichtung ablaufen:
1. Schritt: Betätigung des Handhebels beziehungsweise des Bedienelements und Brechen der Ampulle beziehungsweise Ampullen, Auslaufen der Monomerflüssigkeit innerhalb von 1 bis 2 Sekunden in den Pumpraum, der mit einem Leitungsmittel (der Fluidverbindung) und einer Düse verbunden ist, wobei die Düse mit der Öffnung in den Innenraum der Kartusche zeigt, beim Anschlagpunkt des Hebels Einrasten einer elastischen Stange in ein Gegenrastmittel des Hebels, wobei die Stange in einen ersten Teil und einen zweiten Teil gegabelt ist;
2. Schritt: Rückwärtsbewegung des Handhebels beziehungsweise des Bedienelements in die Ausgangsposition, wobei durch den ersten Teil der elastischen Stange ein Vakuumkolben und ein Pumpkolben in einem Hohlzylinder axial bewegt werden und ein Unterdrück hinter dem Vakuumkolben im Hohlzylinder entsteht und ein Druck hinter dem Pumpkolben auf die Monomerflüssigkeit im Pumpraum im Hohlzylinder ausgeübt wird, Weiterleitung des Unterdrucks über ein Rückschlagventil, ein Leitungsmittel zum Vakuumanschluss in den Innenraum der Kartusche, Ausbildung eines Unterdrucks im Innenraum der Kartusche, Eindrücken der Monomerflüssigkeit aus dem Pumpraum durch die Fluidverbindung und eine Düse in die Kartusche und paralleles Ansaugen der Monomerflüssigkeit aus dem Pumpraum in die Kartusche, Bewegung des zweiten Teils der elastischen Stange, die mit einer ersten Hülse drehbar verbunden ist, die mindestens eine äußerte Nocke besitzt, die in einem Steilgewinde einer zweiten Hülse beweglich angeordnet ist, dass ein elastischer Rührstab (als Mischwelle) mit der ersten Hülse fest verbunden ist, so dass bei axialer Bewegung der ersten Hülse durch die (beziehungsweise in der) zweiten Hülse die erste Hülse durch Eingriff der Nocke in das Steilgewinde gedreht wird, wodurch der Rührstab und rotiert und sich axial in der Kartusche bewegt;
3. Schritt: Weitere Betätigung des Handhebels beziehungsweise des Bedienelements, Bewegung der ersten Hülse in der zweiten Hülse, axiale Bewegung des Rührstabs in der Kartusche unter Drehung um die Längsachse;
4. Schritt: Wiederholung des 2. und 3. Schritts bis der Zementteig homogen gemischt ist;
5. Schritt: Ablösen der Kartusche beziehungsweise des Kartuschensystems durch Herausschrauben und Herausziehen des Mischstabs (der Mischwelle) mit dem Mischelement, unter Umklappen der Mischelemente (der Mischflügel der Mischeinrichtung).

Der wesentliche Vorteil der Erfindung ist, dass ein Prepack-Mischsystem vorgeschlagen wird, das in einfachster Weise, ohne spezielle Schulungsmaßnahmen, durch den medizinischen Anwender durch einfach manuelle Betätigung genutzt kann, um innerhalb von wenigen Sekunden (etwa innerhalb von 40 Sekunden) einen Polymethylmethacrylat-Knochenzementteig herzustellen. Vorteilhaft ist weiterhin, dass auf Grund der maximal vereinfachten Bedienung Anwenderfehler minimiert werden und dadurch die Patientensicherheit verbessert wird.

Die erfindungsgemäße Vakuummischvorrichtung kann im Wesentlichen kostengünstig mit einfachen, durch Kunststoffspritzgießen herzustellenden Kunststoffteilen realisiert werden. Der besondere Vorteil der erfindungsgemäßen Vorrichtung besteht darin, dass die Vorrichtung ohne äußere Hilfsmittel, wie durch Druckluft angetriebene Vakuumpumpen und Druckpumpen und ohne Vakuumschläuche, und ohne Energiequellen, wie Druckluft oder Batterien, betrieben werden kann. Die erfindungsgemäße Vakuummischvorrichtung ist autonom verwendbar und kann selbst unter einfachsten beziehungsweise schwierigsten Operationsbedingungen verwendet werden. Mit der erfindungsgemäßen Vakuummischvorrichtung wird ein geschlossenes Full-Prepack-Vakuumzementiersystem für preissensitive Märkte zur Verfügung gestellt.

Im Folgenden werden weitere Ausführungsbeispiele der Erfindung anhand von neun schematisch dargestellten Figuren erläutert, ohne jedoch dabei die Erfindung zu beschränken. Dabei zeigt:
- Figur 1:: eine schematische perspektivische Ansicht einer erfindungsgemäßen Vakuummischvorrichtung;
- Figur 2:: eine schematische perspektivische Ansicht der Vakuummischvorrichtung nach Figur 1 mit geöffnetem Gehäuse;
- Figur 3:: eine schematische perspektivische Ansicht der Vakuummischvorrichtung nach Figur 1 und 2 mit dem auf der anderen Seite als in Figur 2 geöffneten Gehäuse;
- Figur 4:: eine schematische perspektivische Querschnittansicht der Vakuummischvorrichtung nach den Figuren 1 bis 3 im Ausgangszustand;
- Figur 5:: eine schematische Querschnittansicht der Vakuummischvorrichtung nach den Figuren 1 bis 4 im Ausgangszustand;
- Figur 6:: eine schematische Querschnittansicht der Vakuummischvorrichtung nach den Figuren 1 bis 5 mit einer Schnittebene senkrecht zu dem Schnitt der Figuren 4 und 5;
- Figur 7:: eine schematische Querschnittansicht der Vakuummischvorrichtung nach den Figuren 1 bis 6 während der Bedienung mit aufgebrochener Glasampulle;
- Figur 8:: eine schematische Querschnittansicht der Vakuummischvorrichtung nach den Figuren 1 bis 7 während der Bedienung mit eingerastetem Kabel oder eingerasteter Stange; und
- Figur 9:: eine schematische Querschnittansicht der Vakuummischvorrichtung nach den Figuren 1 bis 8 während der Bedienung beim Pumpen und Mischen.

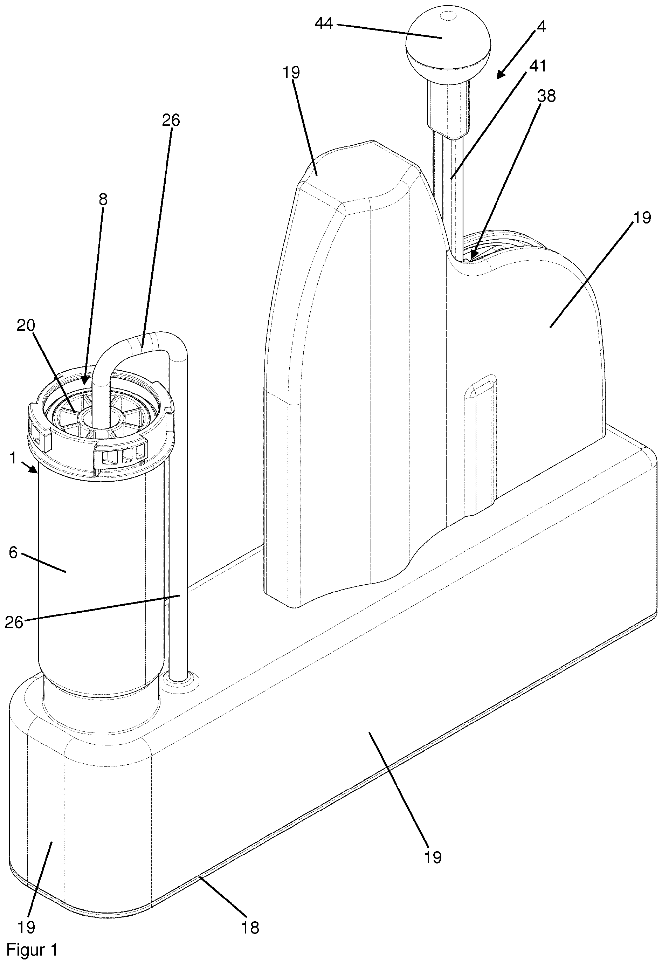

Die Figuren 1 bis 9 zeigen verschiedene Ansichten einer erfindungsgemäßen Vakuummischvorrichtung vor und während des Betriebs. Die Vakuummischvorrichtung besteht im Wesentlichen aus fünf Teilen, nämlich einem Kartuschensystem 1, einem Flüssigkeitsbehälter 2, einer manuell anzutreibenden kombinierten Druck-Vakuumpumpe 3, einem Bedienelement 4 und einer Öffnungseinrichtung 5.

Zentraler Bestandteil des Kartuschensystems 1 ist eine Kartusche 6 mit einem zylindrischen Innenraum, die an ihrer Oberseite durch einen zweiteiligen Austragskolben 8 verschlossen ist, der im zylindrischen Innenraum der Kartusche 6 in Längsrichtung beweglich angeordnet ist. Die Kartusche 6 hat also einen zylindrischen Innenraum mit kreisförmiger Grundfläche. Die Kartusche 6 enthält ein Zementpulver 9 als Ausgangskomponente für einen Knochenzement.

Im Innenraum der Kartusche 6 ist ferner eine Mischeinrichtung 10 mit zwei oder mehr Mischflügeln 10 angeordnet, wobei die Mischeinrichtung 10 drehbar und in Längsrichtung verschiebbar im Innenraum der Kartusche 6 gelagert ist und die an einer Mischwelle 12 oder an einem Kabel 12 befestigt ist, die oder das drehbar und in Längsrichtung verschiebbar durch eine Durchführung in der Unterseite der Kartusche 6 in den Innenraum der Kartusche 6 geführt ist. Die Durchführung ist hierzu druckdicht und gasdicht ausgeführt. Die Mischwelle 12 kann auch als flexible Stange 12 ausgeführt werden.

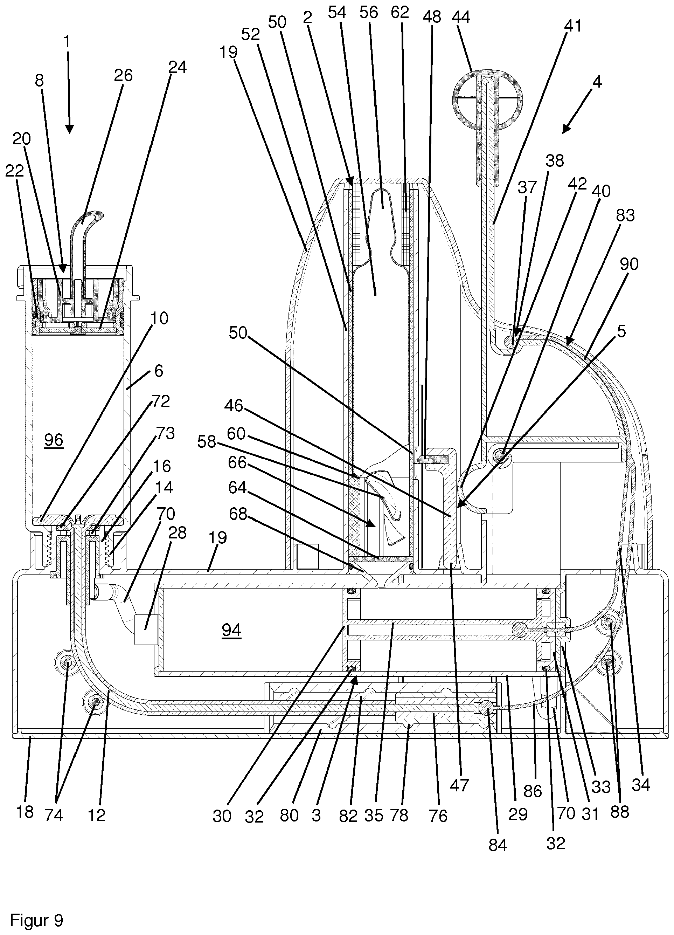

Das Kartuschensystem 1 ist mit dem Flüssigkeitsbehälter 2 und der Druck-Vakuumpumpe 3 über ein Fußteil 18 und ein Gehäuse 19 verbunden. Der Flüssigkeitsbehälter 2, die Druck-Vakuumpumpe 3, ein Teil des Bedienelements 4 und die Öffnungseinrichtung 5 sind von dem Gehäuse 19 umschlossen, wobei ein Teil des Bedienelements 4 aus dem Gehäuse 19 herausragt, während das Kartuschensystem 1 auf das Gehäuse 19 aufgeschraubt ist. Die Kartusche 6 endet an ihrer Unterseite in einem Stutzen mit einem Innengewinde 14, das auf ein Außengewinde 16 an einem Stutzen des Gehäuses 19 geschraubt ist. Das Fußteil 18 bildet dabei den Standfuß 18 der kompakten Vakuummischvorrichtung. Die Kartusche 6 ist dadurch von dem Gehäuse 19 und damit dem Rest der Vakuummischvorrichtung lösbar. Wenn der Knochenzement 96 (siehe Figur 9) mit der Vakuummischvorrichtung im Innenraum der Kartusche 6 fertig gemischt ist, kann also die Kartusche 6 vom Gehäuse 19 abgeschraubt werden und in das Innengewinde 14 kann ein Austragsrohr (nicht gezeigt) eingeschraubt werden, durch das der fertige Knochenzementteig 96 (siehe Figur 9) durch Vortreiben des Austragskolbens 8 in Richtung des Innengewindes 14 ausgetrieben werden kann. Im Austragsrohr kann ein statischer Mischer vorgesehen sein, der eine zusätzliche Durchmischung des Knochenzementteigs 96 bewirkt.

Der zweiteilige Austragskolben 8 hat einen Dichtungskolben 20 und einen Sterilisationskolben 22. Der Sterilisationskolben 22 weist eine Membran oder Porenscheibe 24 auf, die für ein sterilisierendes Gas durchlässig ist aber für das Zementpulver 9 nicht durchlässig ist. Der Sterilisationskolben 22 wird nach dem Einfüllen des Zementpulvers 9 in die Kartusche 6 eingesetzt und schließt den Innenraum der Kartusche 6 nach außen ab. Anschließend kann der Inhalt der Kartusche 6 durch die gasdurchlässige Membran oder Porenscheibe 24 mit Ethylendioxid sterilisiert werden.

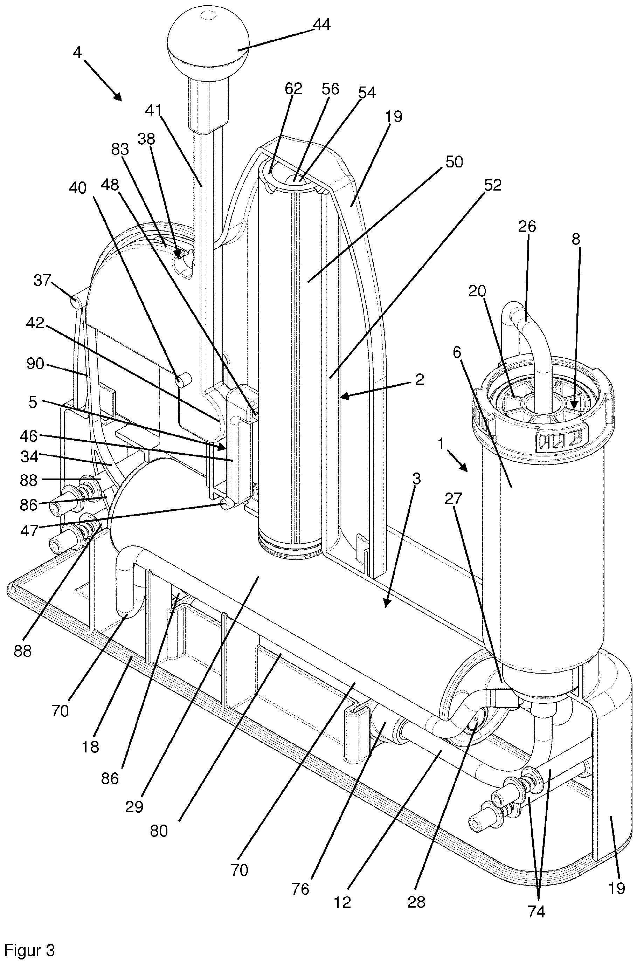

Der Dichtungskolben 20 kann in den Sterilisationskolben 22 gedrückt und mit diesem gas- und druckdicht verbunden werden. Die aneinander befestigten Kolbenteile 20, 22 bilden dann zusammen den Austragskolben 8, mit dem der Inhalt der Kartusche 6 durch die Öffnung im Stutzen mit dem Innengewinde 14 herausgepresst werden kann. Zunächst ist der Sterilisationskolben 22 auf der mit der Öffnung in dem Stutzen mit dem Innengewinde 14 gegenüberliegenden Seite (in den Figuren 3 bis 5 und 7 bis 9 oben) arretiert, wobei die Arretierung lösbar ist. Durch die Arretierung wird verhindert, dass sich der Sterilisationskolben 22 während der Sterilisation des Innenraums der Kartusche 6 sowie des Zementpulvers 9 ungewollt bewegt.

Über die Mischwelle 12 beziehungsweise das Kabel 12 können die Mischflügel 10 im Inneren der Kartusche 6, also im Innenraum der Kartusche 6 gedreht werden und in Längsrichtung der Kartusche 6 bewegt werden.

In dem Dichtungskolben 20 ist eine Durchführung vorgesehen, die an eine Verbindungsleitung 26 in Form einer flexiblen Vakuumleitung 26 angeschlossen ist. Der Dichtungskolben 20 schließt ansonsten druckdicht mit der Kartusche 6.

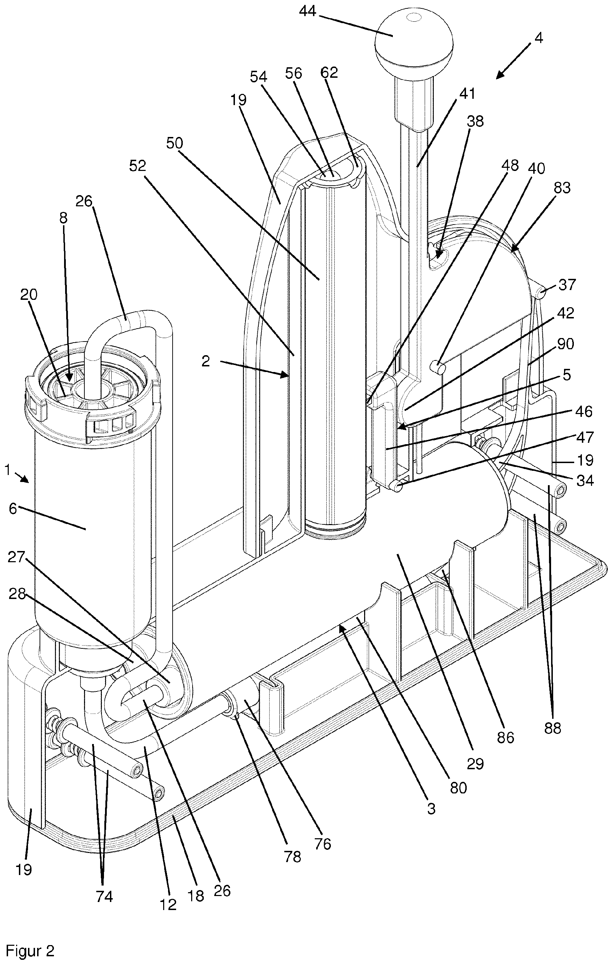

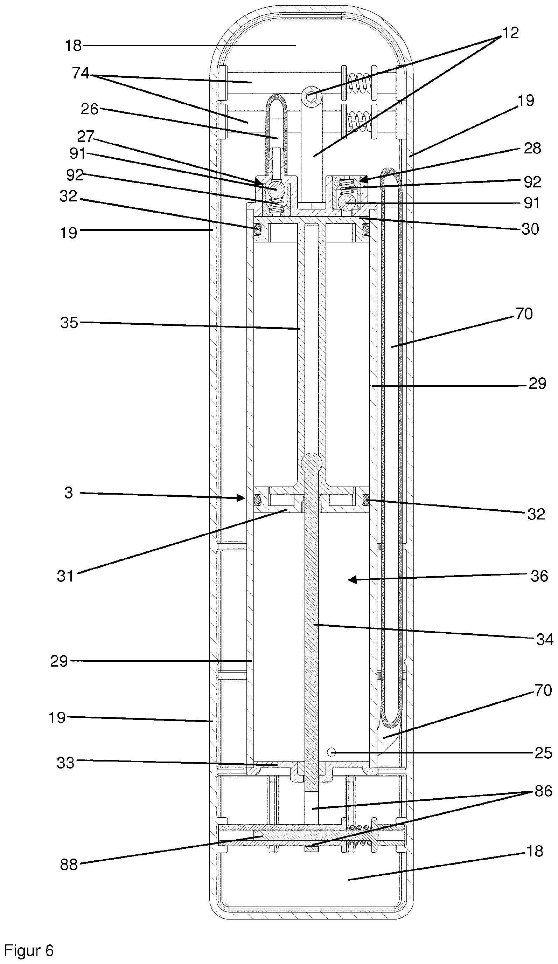

Die kombinierte Druck-Vakuumpumpe 3 umfasst eine Vakuumpumpe 3, der durch den vorderen Teil der kombinierten Druck-Vakuumpumpe 3 gebildet ist (in den Figuren 2, 4, 5 und 7 bis 9 links, in Figur 3 rechts und in Figur 6 oben), und eine Druckpumpe 3, der durch den hinteren Teil der kombinierten Druck-Vakuumpumpe 3 gebildet ist (in den Figuren 2, 4, 5 und 7 bis 9 rechts, in Figur 3 links und in Figur 6 unten).

Die Vakuumleitung 26 mündet über ein erstes Rückschlagventil 27 in die Vakuumpumpe 3, so dass nur eine Strömung in Richtung in die Vakuumpumpe 3 hinein möglich ist. Die Vakuumpumpe 3 ist zudem über ein zweites Rückschlagventil 28 mit der Umgebung verbunden, so dass ein in der Vakuumpumpe 3 entstehender Überdruck über das zweite Rückschlagventil 28 abgeblasen werden kann. Details zu den Rückschlagventilen 27, 28 sind in Figur 6 dargestellt und in der Beschreibung weiter unten ausgeführt. An dem zweiten Rückschlagventil 28 kann optional ein Filter (nicht gezeigt) vorgesehen sein, mit dem Methacrylatdämpfe oder andere störende chemische Substanzen aus dem ausströmenden Gas herausgefiltert werden.

Die Druck-Vakuumpumpe 3 ist mit einem gemeinsamen stabilen Hohlzylinder 29 aufgebaut, der die beiden Teile der Druck-Vakuumpumpe 3, also die Vakuumpumpe 3 und die Druckpumpe 3 verbindet. Der Hohlzylinder 29 ist über einen Vakuumkolben 30 und über einen Pumpkolben 31 druckdicht in drei Teile getrennt. Hierzu weisen der Vakuumkolben 30 und der Pumpkolben 31 umlaufende Dichtungen 32 auf, die mit der Innenwand des Hohlzylinders 29 abschließen. Der Vakuumkolben 30 und der Pumpkolben 31 sind mit einem Kabel 34 oder einer flexiblen Stange 34 aus einem stabilen elastischen Kunststoff oder aus einem Metall wie Stahl verbunden, das durch eine Durchführung in einem rückseitigen Verschluss 33 führt. Der Vakuumkolben 30 und der Pumpkolben 31 sind über ein Rohr 35 oder eine Stange 35 fest miteinander verbunden, so dass der Abstand des Vakuumkolbens 30 zu dem Pumpkolben 31 in der Druck-Vakuumpumpe 3 feststeht.

Auf der Vorderseite ist die Vakuumpumpe 3 durch einen vorderseitigen Verschluss verschlossen, in dem sich die Anschlüsse für die Rückschlagventile 27, 28 befinden. Der Verschluss 33 ist auf der den Rückschlagventilen 27, 28 gegenüberliegenden Seiten des Hohlzylinders 29 angeschlossen. Die Vakuumleitung 26 ist bis zu der Vakuumpumpe 3 geführt, so dass die Durchführung im Dichtungskolben 20 über die Vakuumleitung 26 druckdicht mit der Vakuumpumpe 3 genauer mit einem Vakuumpumpraum 94 (siehe Figur 9) der Vakuumpumpe 3 verbunden ist. Der Vakuumpumpraum 94 ist durch die Innenwände des Hohlzylinders 29, durch den vorderseitigen Verschluss und durch den Vakuumkolben 30 begrenzt. Bei der Druckpumpe 3 ist ein Druckpumpraum 36 durch den Hohlzylinder 29, durch den Pumpkolben 31 und durch den Verschluss 33 begrenzt. Der Raum zwischen dem Vakuumkolben 30 und dem Pumpkolben 31 wird außer zum Verhindern des Eindringens von Monomerflüssigkeit in den Vakuumpumpraum 94 nicht genutzt.

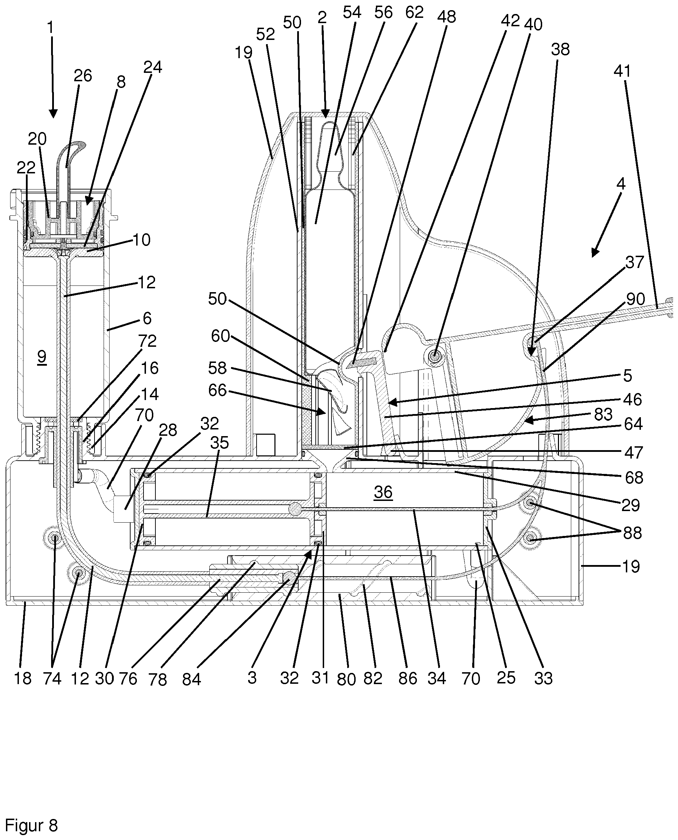

Das Kabel 34 ist mit einem Rastmittel 37 verbunden, das mit einem Gegenrastmittel 38 rasten kann (siehe Figuren 8 und 9). Das Gegenrastmittel 38 ist Teil des Bedienelements 4, das als ein um eine Achse 40 drehbarer beziehungsweise schwenkbarer Hebel 4 aufgebaut ist. Der Hebel 4 umfasst zwei Hebelarme 41, 42, die sich von der Achse 40 aus in unterschiedliche Richtungen erstrecken. Das eigentliche Bedienteil des Hebels 4 bildet der erste Hebelarm 41, der in einem Griff 44 endet, der von außen manuell bedienbar ist. Der erste Hebelarm 41 ragt also aus dem Gehäuse 19 heraus und der Griff 44 ist außerhalb des Gehäuses 19 angeordnet und kann vom Anwender der Vakuummischvorrichtung manuell bedient werden. Dabei kann über den ersten Hebelarm 41 eine beträchtliche Kraft ins Innere der Vakuummischvorrichtung übertragen werden, die zum Antreiben der Öffnungseinrichtung 5, der Vakuumpumpe 3, der Druckpumpe 3, beziehungsweise der Druck-Vakuumpumpe 3, und der Mischeinrichtung 10 ausreicht und erfindungsgemäß angewendet wird.

Der zweite Hebelarm 42 drückt beim Schwenken des Hebels 4 beziehungsweise beim nach unten drücken des Hebels 4 auf die Öffnungseinrichtung 5 und treibt diese an. Dazu ist die Öffnungseinrichtung 5 mit einem Hebel 46 aufgebaut, der um eine Achse 47 drehbar beziehungsweise schwenkbar gelagert ist. An dem der Achse 47 gegenüberliegenden Ende des Hebels 46 ist ein Einsatz mit einer Kante 48 vorgesehen, die an der Aufnahme 2 beziehungsweise dem Flüssigkeitsbehälter 2 anliegt.

Der Flüssigkeitsbehälter 2 beziehungsweise die Aufnahme 2 umfasst einen inneren elastischen Einsatz 50, der beispielsweise aus einem Gummi bestehen kann, und einen starren Hohlzylinder 52 aus einem Kunststoff wie Plastik. In der Aufnahme 2 beziehungsweise dem Flüssigkeitsbehälter 2 ist eine Glasampulle 54 enthaltend eine Monomerflüssigkeit eingesteckt. Die Monomerflüssigkeit bildet mit dem Zementpulver 9 aus der Kartusche 6 einen Knochenzementteig 96, wenn sie miteinander durchmischt werden. Die Innenwandungen des elastischen Einsatzes 50 liegen an der Glasampulle 54 an. Die Glasampulle 54 weist einen Ampullenkopf 56 und einen dem Ampullenkopf 56 gegenüberliegenden Ampullenboden 58 auf. Die Glasampulle 54 sitzt mit dem Ampullenboden 58 auf einer Auflage 60 auf, die als Absatz 60 des elastischen Einsatzes 50 ausgeformt ist. Auf der Oberseite drückt ein Hohlzylinder 62 aus einem gasdurchlässigen Schaumstoff, der an der Innenseite des Gehäuses 19 befestigt ist, die Glasampulle 54 auf die Auflage 60. Zwischen dem Hohlzylinder 52 und dem Gehäuse 19 sind Öffnungen vorgesehen, durch die Luft oder Gas aus der Umgebung der Aufnahme 2 und der Vakuummischvorrichtung in die Aufnahme 2 nachströmen kann. Dadurch kann die Monomerflüssigkeit leichter aus der Aufnahme 2 abfließen. Die Öffnungen zwischen dem Hohlzylinder 52 und dem Gehäuse 19 sind derart gestaltet, dass sie in der Zylindermantelwand des Hohlzylinders 52 angrenzend an die obere Grundfläche des Hohlzylinders 52 angeordnet sind. Der Hohlzylinder 52 liegt also nur bereichsweise an dem Gehäuse 19 an. Dazwischen kann Luft ins Innere der Aufnahme 2 nachströmen.

Im Bereich des Ampullenbodens 58 beziehungsweise der Auflage 60 weist der Hohlzylinder 52 eine Ausnehmung auf, innerhalb der die Kante 48 an dem elastischen Einsatz 50 anliegt, so dass der Ampullenboden 58 mit der Öffnungseinrichtung 5 abgebrochen und damit die Glasampulle 54 geöffnet werden kann. Der Ampullenkopf 56 der Glasampulle 54 wird üblicherweise zum Öffnen der Glasampulle 54 abgebrochen. Da die Glasampulle 54 am Hals dünn ausgeführt ist, führt dies jedoch dazu, dass die Monomerflüssigkeit aus der Glasampulle 54 nur langsam auslaufen kann und daher der Anwender warten muss, bis er die nächsten Schritte zum Bedienen der Vakuummischvorrichtung durchführen kann. Dies ist bei dem weitgehend automatisierten Verfahren, das durch Bedienen des Hebels 4 beziehungsweise des Bedienelements 4 angetrieben wird nicht geeignet, da so nicht sichergestellt werden könnte, dass die Monomerflüssigkeit aus der Glasampulle 54 schon zur Verfügung steht, wenn die Vakuumpumpe 3 über das Bedienelement 4 angetrieben wird.

Die Glasampulle 54 steckt in dem Einsatz 50 aus dem verformbaren Material. Der Einsatz 50 bildet zusammen mit dem Hohlzylinder 52 die wesentlichen Teile der Aufnahme 2 für die Glasampulle 54. Die Glasampulle 54 kann aufgrund des Absatzes 60 nur bis zum Ampullenboden 58 in den Einsatz 50 des Flüssigkeitsbehälters 2 eingeschoben werden.

Der Flüssigkeitsbehälter 2 weist eine seitliche Öffnung auf, in der der Einsatz 50 eine verformbare Seitenwand bildet. An dieser Stelle kann die Glasampulle 54 geöffnet beziehungsweise aufgebrochen werden, indem ein Druck durch die verformbare Seitenwand 50 auf die Glasampulle 54 knapp oberhalb des Ampullenbodens 58 wirkt. Wenn der Ampullenboden 58 der Glasampulle 54 abgebrochen beziehungsweise die Glasampulle 54 geöffnet wird, kann die Monomerflüssigkeit aus der geöffneten Glasampulle 54 im vollen Querschnitt ausfließen, so dass die Monomerflüssigkeit schnell im vollen Umfang zur Weiterverarbeitung innerhalb der Vakuummischvorrichtung zur Verfügung steht.

Um die verformbare Seitenwand 50 zu verformen und dadurch die Glasampulle 54 aufzubrechen, wird der Hebel 42 verwendet, der über den Hebel 4 bedienbar ist und der um die Achse 40 gedreht werden kann. Der Hebel 4 ist schwenkbar beziehungsweise drehbar um die Achse 40 gegen das Gehäuse 19 gelagert. Die Achse 40 teilt den Hebel 4 in einen langen Hebelarm 41, an dem der Griff 44 befestigt ist und einen kurzen Hebelarm 42 der innerhalb des Gehäuses 19 angeordnet ist. Zu Beginn kann der lange Hebelarm 41 nur von dem Flüssigkeitsbehälter 2 weg bewegt werden und nicht auf diesen zu, da der lange Hebelarm 41 oben an der Öffnung des Gehäuses 19 anliegt und so eine weitere Bewegung in diese Richtung unterbindet.

Der kurze Hebelarm 42 des Hebels 4 liegt auf seiner dem Flüssigkeitsbehälter 2 zugewandten Seite an dem Hebel 46 der Öffnungseinrichtung 5 an, der über ein Gelenk 47 beziehungsweise die Achse 47 drehbar um die Achse 47 mit dem Fußteil 18 beziehungsweise dem Gehäuse 19 der Vakuummischvorrichtung verbunden ist. Dieser Hebel 46 der Öffnungseinrichtung 5 ist innerhalb des Gehäuses 19 angeordnet. Das freie Hebelende des Hebels 46 im Gehäuse 19 kann mit dem kurzen Hebelarm 42 bewegt werden. An der Spitze des freien Hebelendes ist die Kante 48 befestigt, die an der verformbaren Seitenwand 50 anliegt. Die Achse 47 des Hebels 46 ist dabei so angeordnet, dass sich das freie Hebelende und damit die Kante 48 in Richtung der verformbaren Seitenwand 50 und in Richtung des Fußteils 18 bewegt. Dadurch wird erreicht, dass die Kraft, die von der Kante 48 durch die verformbare Seitenwand 50 auf die Glasampulle 54 ausgeübt werden kann, die Glasampulle 54 auch leicht in Richtung des Absatzes 60 presst und somit die Glasampulle 50 in die Aufnahme 2 hineindrückt.

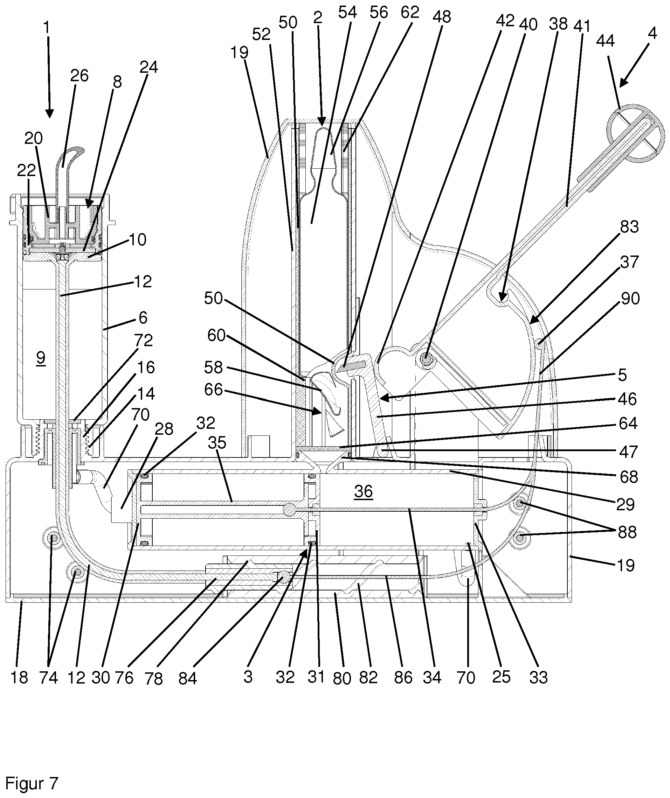

Unterhalb des Absatzes 60 ist ein Sieb 64 und/oder ein Filter 64 angeordnet, mit dem oder mit denen Glassplitter der geöffneten beziehungsweise aufgebrochenen Glasampulle 54 zurückgehalten werden. Der Abstand zwischen dem Absatz 60 und dem Sieb 64 und/oder Filter 64 ist größer als der Außendurchmesser der Glasampulle 54, so dass der abfallende Ampullenboden 58 sich in einem Zwischenraum 66 drehen kann und den Ausfluss der Monomerflüssigkeit aus der geöffneten Glasampulle 54 nicht behindert (siehe Figuren 7 bis 9). Unterhalb des Siebs 64 und/oder Filters 64 ist ein Trichter 68 angeordnet, der in den Druckpumpraum 36 mündet. Die Mündung des Trichters 68 ist dicht bei dem Pumpkolben 31 in seiner Ausgangsstellung angeordnet, so dass der Pumpkolben 31 die Einmündung kurz nach dem Beginn der Bewegung des Pumpkolbens 31 abdeckt und nicht fortwährend Monomerflüssigkeit zurück in die Aufnahme 2 gedrückt wird. Der Druckpumpraum 36 ist über eine Einmündung 25 mit einer Fluidverbindung 70 verbunden, die mit dem Innenraum der Kartusche 6 verbunden ist. Die Vorderseite der Kartusche 6 (in den Figuren 1 bis 5 und 7 bis 9 unten) ist durch das Fußteil 18 und das Gehäuse 19 über die Fluidverbindung 70 mit der Einmündung 25 dicht mit dem Druckpumpraum 36 verbunden.

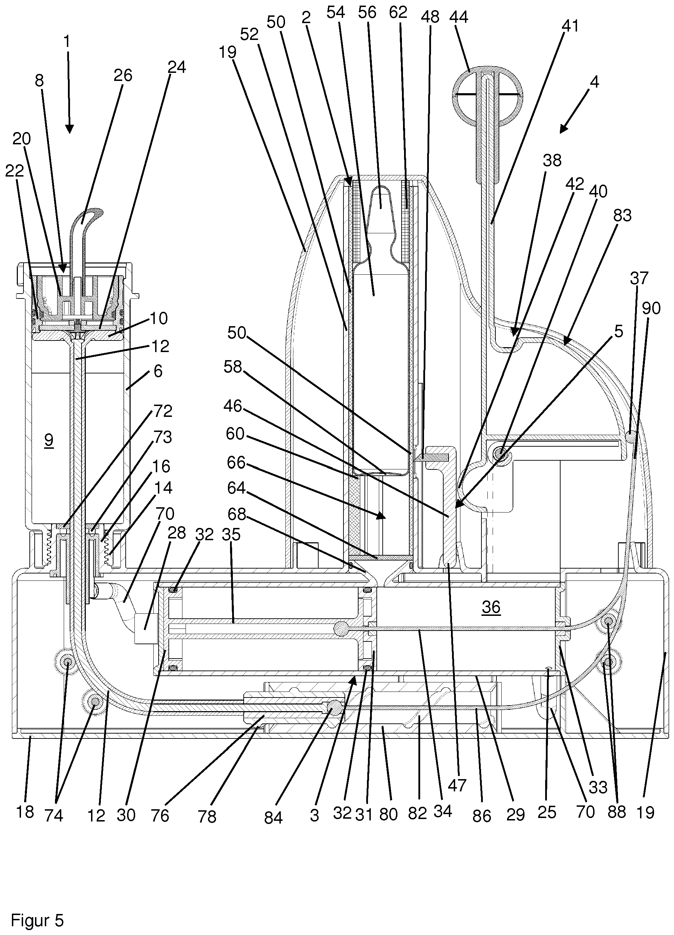

Die Kartusche 6 ist an dem Gehäuse 19 senkrecht lösbar befestigt. Die Fluidverbindung 70 mündet in dem Stutzen mit dem Außengewinde 16 durch einen pulverundurchlässigen aber für die Monomerflüssigkeit durchlässigen Filter 72 in den Innenraum der Kartusche 6. Unterhalb des Filters 72 ist ein ringförmiger Kanal 73 ausgebildet (nur in den Figuren 3, 4 und 9 gekennzeichnet aber auch in den Figuren 5, 7 und 8 zu erkennen), der zum Filter 72 hin offen ist, so dass der ebenfalls ringförmige Filter 72 den ringförmigen Kanal abdeckt. Der ringförmige Kanal 73, in den die Fluidverbindung 70 mündet und der genaugenommen noch zur Fluidverbindung 70 gehört, und der ringförmige Filter 72 umschließen den Durchgang, in dem die Mischwelle 12 beziehungsweise das Kabel 12 in den Innenraum der Kartusche 6 geführt ist. Im Durchgang können dazu Abdichtungen (nicht gezeigt) oder zumindest Abstreifer (nicht gezeigt) vorgesehen sein. Mit dem ringförmigen Kanal 73 wird erreicht, dass die Monomerflüssigkeit durch den Filter 72 um die Mischwelle 12 herum in den Innenraum der Kartusche 6 eingeleitet wird. Es kann am Eingang der Fluidverbindung 70 in den Innenraum der Kartusche 6 auch eine Düse (nicht gezeigt) vorgesehen sein, die die Monomerflüssigkeit im Innenraum beziehungsweise im Zementpulver 9 verteilt.

Der Flüssigkeitsbehälter 2 wird mit dem Gehäuse 19 nach oben verschlossen, nachdem die Glasampulle 54 in den Flüssigkeitsbehälter 2 eingesetzt wurde. Damit die Monomerflüssigkeit ohne Probleme aus der Glasampulle 54 und dem Zwischenraum 66 auslaufen beziehungsweise ablaufen kann, können zusätzlich in dem den Flüssigkeitsbehälter 2 abdeckenden Teil des Gehäuses 19 mehrere Durchgänge (nicht gezeigt) vorgesehen sein, durch die Luft von außerhalb in den Flüssigkeitsbehälter 2 nachströmen kann. Nach dem Aufbrechen der Glasampulle 54 fließt die Monomerflüssigkeit durch den Zwischenraum 66 und den Trichter 68 in den Druckpumpraum 36 und kann durch die Fluidverbindung 70 in den Innenraum der Kartusche 6 gedrückt werden, in dem mit dem Pumpkolben 31 ein Druck auf die Monomerflüssigkeit im Druckpumpraum 36 ausgeübt wird. Gleichzeitig wird ein Unterdruck in dem Innenraum der Kartusche 6 erzeugt, mit dem die Monomerflüssigkeit aus dem Druckpumpraum 36 in den Innenraum der Kartusche 6 gesaugt wird. Dieser Unterdruck wird mit der Vakuumpumpe 3 erzeugt. Im Innenraum der Kartusche 6 kann die Monomerflüssigkeit dann mit dem Zementpulver 9 mit Hilfe der Mischeinrichtung 10 unter Vakuum beziehungsweise unter Unterdruck gemischt werden, um den Knochenzement 96 beziehungsweise einen Knochenzementteig 96 zu erzeugen.

Die Mischeinrichtung 10 wird zum Durchmischen des Inhalts des Innenraums der Kartusche 6 verwendet. Das Kabel 12 beziehungsweise die Mischwelle 12, über die die Mischeinrichtung 10 im Innenraum gedreht wird und in Längsrichtung des Innenraums auf und ab bewegt wird, wird über Stifte 74 beziehungsweise Umlenkrollen 74 in Richtung eines Zylinders 76 umgelenkt. Die Umlenkrollen 74 können mit federnd gelagerten Röhrchen beziehungsweise Umlenkhülsen aufgebaut werden. Die Federn dienen dabei lediglich der Fixierung der Umlenkrollen 74 beziehungsweise Umlenkhülsen. Das Kabel 12 beziehungsweise die Mischwelle 12 ist starr mit dem Zylinder 76 verbunden. Der Zylinder 76 weist an der Außenseite ein steiles Außengewinde 78 auf. Der Zylinder 76 ist in einer Hülse 80 mit einem zum Außengewinde 78 passenden Innengewinde 82 angeordnet. Bei einer Bewegung des Zylinders 76 in der Hülse 80 in Längsrichtung (der Zylinderachse) wird die Mischeinrichtung 10 über das Kabel 12 beziehungsweise die Mischwelle 12 also in Längsrichtung des Innenraums der Kartusche 6 bewegt und gleichzeitig aufgrund der Gewinde 78, 82 um die Mischwelle 12 gedreht und damit der Inhalt des Innenraums durchmischt. Alternativ zum Außengewinde 78 an der Hülse kann auch ein oder mehrere Vorsprünge beziehungsweise eine oder mehrere Nocken 78 vorgesehen sein, die in dem Innengewinde 82 laufen und so den Zylinder 76 in der Hülse 80 drehen.

Der Zylinder 76 ist über ein Kugelgelenk beziehungsweise einen Kugelgelenkkopf 84 mit einem starren Kabel 86 oder einer flexiblen Stange 86, das analog dem Kabel 34 beziehungsweise der flexiblen Stange 34 für die Druck-Vakuumpumpe 3 aufgebaut ist, verbunden. Der Kugelgelenkkopf 84 kann sich also innerhalb einer Aufnahme für den Kugelgelenkkopf 84 des Zylinders 76 bewegen und darin drehen. Hierdurch wird ermöglicht, dass bei einer Bewegung des Kabels 86 gleichzeitig eine Drehung des Zylinders 76 in der Hülse 80 erzwungen wird. Das Kabel 86, das mit dem Zylinder 76 verbunden ist und das Kabel 34, das mit dem Pumpkolben 31 der Druck-Vakuumpumpe 3 verbunden ist, sind miteinander verbunden, wobei beide über Stifte 88 beziehungsweise Umlenkrollen 88 positioniert werden. Die Umlenkrollen 88 sind analog den Umlenkrollen 74 aufgebaut. Ebenso kann die Verbindung des Kabels 34 zum Pumpkolben 31 mit einem Kugelgelenk aufgebaut werden. Die beiden Kabel 34, 86 oder flexiblen Stangen 34, 86 schließen sich zu einem Kabel 90 oder einer flexiblen Stange 90 zusammen, das nach oben zum Hebel 4 beziehungsweise zum Bedienelement 4 geführt ist, wobei das Kabel 90 oder die flexible Stange 90 dort in dem Rastmittel 37 endet. Auch das Kabel 90 ist analog dem Kabel 34 für die Druck-Vakuumpumpe 3 aufgebaut, beziehungsweise die flexible Stange 90 analog der flexiblen Stange 34 für die Druck-Vakuumpumpe 3. Die gegabelt verbundenen Kabel 34, 86, 90 oder die gegabelte Stange 34, 86, 90 können aus einem Kunststoff durch Spritzguss gefertigt sein, beziehungsweise das gemeinsame gegabelte Kabel 34, 86, 90 oder die gegabelte Stange 34, 86, 90 kann aus einem Kunststoff durch Spritzguss gefertigt sein. Das Rastmittel 37 am Ende des Kabels 90 ist dabei so gelagert und derart vorgespannt, dass es in das Gegenrastmittel 38 greift und mit diesem rastet, wenn der Hebel 4 weit genug gedreht beziehungsweise geschwenkt wird, beziehungsweise wenn das Gegenrastmittel 38 auf die Höhe des Rastmittels 37 geschwenkt wird.

Der maximale Hub, der durch eine als Evolvente 83 geformte Abrundung am Hebel 4 bestimmt ist, indem das Kabel 90 oder die flexible Stange 90 nach erfolgter Rastung auf die Evolvente 83 aufgezogen wird, reicht dazu aus, dass der Innenraum der Kartusche 6 in voller Länge von der Mischeinrichtung 10 durchfahren werden kann. Dies ist in den Figuren 3, 4, 5, 7 und 8 im Vergleich mit Figur 9 zu erkennen, da beim vollständigen Hub des Hebels 4, der in Figur 9 dargestellt ist, die Mischeinrichtung 10 beziehungsweise die Mischflügel 10 an der Vorderseite des Innenraums der Kartusche 6 auf dem Filter 72 anliegt, während ohne Hub, wie in Figur 8 dargestellt, die Mischeinrichtung 10 beziehungsweise die Mischflügel 10 auf der Rückseite des Innenraums der Kartusche 6 an dem Austragskolben 8, beziehungsweise dem Sterilisationskolben 22 und der Porenscheibe 24 anliegen. Hierdurch wird eine vollständige Durchmischung des Innenraums der Kartusche 6 mit der Mischeinrichtung 10 ermöglicht. Das Gegenrastmittel 38 ist hierzu an dem bezüglich der Zugrichtung der Kabel 90, 34, 86 oder der flexiblen Stange 90, 34, 86 abgewandten Ende der Evolvente 83 angeordnet, damit das Kabel 90 oder die flexible Stange 90 über einen weiten Bereich der Evolvente 83 aufgezogen werden kann.

Anstatt das Kabel 34 oder die flexible Stange 34 der Druck-Vakuumpumpe 3 und das Kabel 86 oder die flexible Stange 86 zum Zylinder 76 miteinander zu dem Kabel 90 oder der flexiblen Stange 90, an dem das Rastmittel 37 angeordnet ist, zu verbinden, kann genauso gut jedes der Kabel 34, 86 oder jede der flexiblen Stangen 34, 86 ein eigenes Rastmittel aufweisen, das in das Gegenrastmittel 38 oder das zwei unterschiedliche Gegenrastmittel am Ende der Evolvente 83 beziehungsweise dem Hebel 4 greift und mit diesem beziehungsweise diesen rastet.

In einer alternativen Ausführung einer erfindungsgemäßen Mischvorrichtung kann das Kabel 12 direkt mit dem Kabel 86 verbunden sein, beziehungsweise die beiden Kabel 12, 86 als ein gemeinsames durchgehendes Kabel ausgeführt sein, oder die flexible Stange 12 mit der flexiblen Stange 86 einteilig ausgeführt sein. Der Zylinder 76, die Hülse 80 sowie die Gewinde 78, 82 sind dann überflüssig und nicht vorhanden. Dies führt dann dazu, dass die Mischeinrichtung 10 nicht mehr durch die Mischwelle 12 im Innenraum der Kartusche 6 gedreht wird. Eine Durchmischung des Innenraums der Kartusche 6 wird dann nur noch durch die Auf- und Abwärtsbewegung der Mischeinrichtung 10 in Längsrichtung erreicht. Durch eine geeignete Neigung der Mischflügel 10 oder einiger Mischflügel 10 und/oder durch eine Führung zumindest eines Vorsprungs (nicht gezeigt) an der Mischeinrichtung 10 in wenigstens einer spiralförmigen Nut (nicht gezeigt) in der Innenwand der Kartusche 6 kann auch auf andere Weise eine Drehung der Mischeinrichtung 10 in der Kartusche 6 erzwungen werden, sofern nicht einfach auf die Drehung der Mischeinrichtung 10 in der Kartusche 6 verzichtet wird.

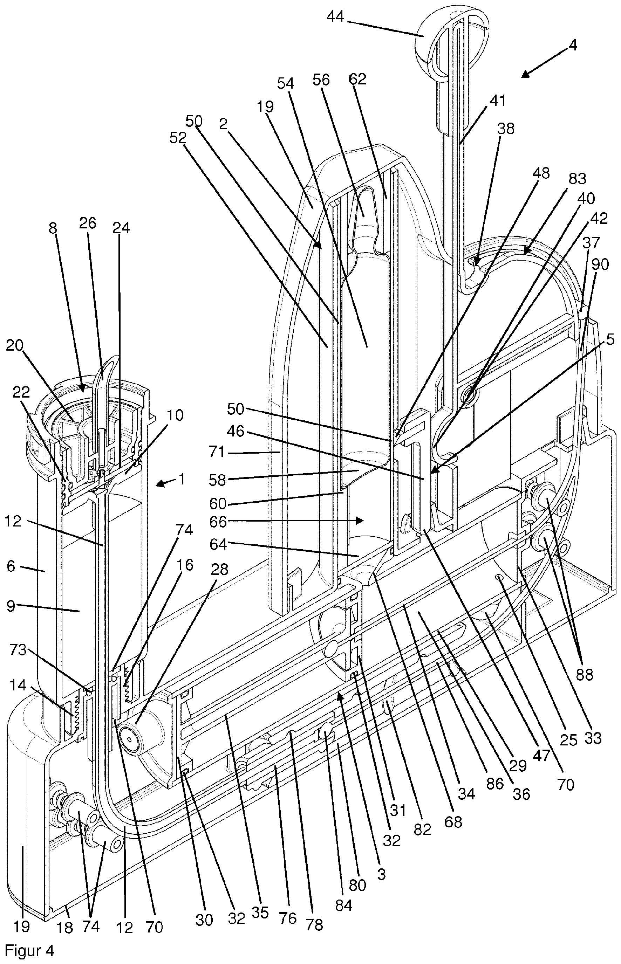

Figur 6 zeigt eine schematische Querschnittansicht der Vakuummischvorrichtung nach den Figuren 1 bis 5 und 7 bis 9 mit einer Schnittebene senkrecht zu dem Schnitt der Figuren 4 und 5 sowie 7 bis 9. Darin ist unter anderem ein beispielhafter Aufbau der Rückschlagventile 27, 28 erläutert. Die Rückschlagventile 27, 28 sind mit Kugeln 91 aufgebaut, die von Federn 92 auf einen Kugelsitz gedrückt werden, in dem sich die Verbindung zur Vakuumleitung 26 beziehungsweise zur Umgebung der Vakuumpumpe 3 befindet. Wenn die Kugeln 91 auf den Kugelsitz gedrückt sind, sind diese Verbindungen geschlossen. Das Rückschlagventil 27 öffnet sich, wenn in der Vakuumpumpe 3 ein Unterdruck relativ zum Druck in der Vakuumleitung 26 beziehungsweise relativ zum Innenraum der Kartusche 6 entsteht beziehungsweise vorhanden ist, indem der Vakuumkolben 30 in Richtung des Verschlusses 33 bewegt wird, also wenn sich der Vakuumpumpraum 94 (siehe Figur 9) öffnet. Das Rückschlagventil 28 öffnet sich dagegen, wenn im Vakuumpumpraum 94 beziehungsweise in der Vakuumpumpe 3 ein größerer Druck herrscht als in der Umgebung. Ansonsten schließen die Rückschlagventile 27, 28 aufgrund der Federkraft der Federn 92.

Die Vakuummischvorrichtung zeichnet sich erfindungsgemäß durch die Anwendbarkeit des folgenden beispielhaften erfindungsgemäßen Verfahrens aus. Die Monomerflüssigkeit wird in der Aufnahme 2 bereitgestellt, indem die Glasampulle 54 mit der Öffnungseinrichtung 5, wie oben geschildert, aufgebrochen wird. Dazu wird der Hebel 4, der sich ursprünglich in einer senkrechten Position (siehe Figur 1 bis 5) befindet nach unten gedrückt (siehe Figur 7). Während die Monomerflüssigkeit in den Druckpumpraum 36 fließt, wird der Hebel 40 weiter gedreht beziehungsweise geschwenkt, bis das Gegenrastmittel 38 auf die Höhe des Rastmittels 37 gedreht ist und beide miteinander rasten (siehe Figur 8). Durch das Rasten des Rastmittels 37 mit dem Gegenrastmittel 38 können erst jetzt die Vakuumpumpe 3 und die Druckpumpe 3 mit dem Hebel 4 über die Kabel 34, 90 oder die flexible Stange 34, 90 bewegt beziehungsweise angetrieben werden und die Mischeinrichtung 10 mit dem Hebel 4 über die Kabel 86, 90 oder die Stange 86, 90, den Zylinder 76 und das Kabel 12 beziehungsweise die Mischwelle 12 angetrieben werden. Dazu wird der Hebel 4 vom unteren Anschlag weg wieder in die Ausgangsposition (Figuren 2 bis 5) gedreht beziehungsweise geschwenkt.

Die Vakuumpumpe 3 der kombinierten Druck-Vakuumpumpe 3 wird verwendet, indem der Vakuumkolben 30 mit dem Bedienelement 4 über das Kabel 34, 90 oder die flexible Stange 34, 90 von den Rückschlagventilen 27, 28 weg in Richtung des Verschlusses 33 gezogen wird. Dabei öffnet sich im Inneren der Vakuumpumpe 3 der Vakuumpumpraum 94 (siehe Figur 9). Durch die Vergrößerung des Vakuumpumpraums 94 wird im Vakuumpumpraum 94 ein geringer Druck erzeugt. Dies führt zu einer Öffnung des Rückschlagventils 27, so dass Gas aus dem Innenraum der Kartusche 6 durch die Verbindungsleitung 26 in den Vakuumpumpraum 94 gesaugt beziehungsweise gedrückt wird. Aufgrund des so entstehenden Unterdrucks wird die Monomerflüssigkeit aus dem Druckpumpraum 36 durch die Fluidverbindung 70 in den Innenraum der Kartusche 6 gesaugt.

Die Druckpumpe 3 der kombinierten Druck-Vakuumpumpe 3 wird verwendet, indem der Pumpkolben 31 mit dem Bedienelement 4 über das Kabel 34, 90 oder die flexible Stange 34, 90 von den Rückschlagventilen 27, 28 weg in Richtung des Verschlusses 33 gezogen wird. Dabei verkleinert sich im Inneren der Druckpumpe 3 der Druckpumpraum 36. Durch die Verkleinerung des Druckpumpraums 36 wird die Monomerflüssigkeit aus dem Druckpumpraum 36 durch Einmündung 25 und die Fluidverbindung 70, den ringförmigen Kanal 73 und den Filter 74 in den Innenraum der Kartusche 6 gedrückt.

Der Pumpkolben 31 wird bis zum Ende des Hohlzylinders 29 (in den Figuren 3 bis 5 und 7 bis 9 rechts) bewegt. Diese Anordnung ist in Figur 9 gezeigt. Die Volumenzunahme des Vakuumpumpraums 96 kann vorzugsweise dazu ausreichen, um das Gas aus der Vakuumleitung 26, dem Innenraum der Kartusche 6 und der Fluidverbindung 70 zu evakuieren und die Monomerflüssigkeit aus dem Flüssigkeitsbehälter 2 in den Innenraum der Kartusche 6 zu ziehen. Bevorzugt kann der expandierte Vakuumpumpraum 96 zu diesem Zweck größer als die Volumina der Leitungen 26, 70, des Innenraums der Kartusche 6 und des Flüssigkeitsvolumens der Monomerflüssigkeit. Der Vakuumpumpraum 94 kann also erfindungsgemäß bevorzugt größer als der Druckpumpraum 36 sein. Alternativ zur Evakuierung des Innenraums der Kartusche 6 mit einem einzigen Hub des Vakuumkolbens 30 kann der Innenraum der Kartusche 6 aber auch mit mehreren Hüben des Vakuumkolbens 30 durch wiederholtes Bedienen des Hebels 4 (hin und her schwenken des Hebels 4) evakuiert werden. Bevorzugt kann dazu der Pumpkolben 36 nicht mehr in Richtung der Rückschlagventile 27, 28 bewegt werden oder zumindest nicht bis über die Einmündung des Trichters 68 in den Druckpumpraum 36 bewegt werden. Dies kann beispielsweise dadurch erreicht werden, dass der Pumpkolben 31 lösbar mit dem Kabel 34 beziehungsweise der Stange 34 verbunden ist (siehe Figur 6), so dass der Pumpkolben 31 nach erstmaliger Bedienung an dem Verschluss 33 anliegend in einer Endposition verbleibt. Dabei kann der Pumpkolben 31 vorzugsweise die Einmündung 25 in die Fluidverbindung 70 druckdicht abdecken. Dann kann die Vakuumpumpe 3 zur weiteren Evakuierung des Innenraums der Kartusche 6 beim Mischen des Knochenzementteigs 96 mit der Mischeinrichtung 10 verwendet werden. Bei einer fortwährenden Bewegung des Hebels 4 wird also der Vakuumkolben 30 über das Kabel 34 beziehungsweise die Stange 34 hin und her bewegt, während das Kabel 34 beziehungsweise die Stange 34 durch den Verschluss 33 und den am Verschluss anliegenden Pumpkolben 31 gleitet beziehungsweise durchläuft.

Der Teil des Innenraums des Hohlzylinders 29 zwischen dem vorderen Verschluss mit den Rückschlagventilen 27, 28 daran (in den Figuren 3, 4 und 7 bis 9 links, in Figur 5 rechts und in Figur 6 oben) und dem Vakuumkolben 30 bildet den Vakuumpumpraum 94. Ein Unterdruck im Vakuumpumpraum 94 kann damit durch die Vakuumleitung 26 bis in den Innenraum der Kartusche 6 wirken, beziehungsweise ein Gas aus dem Innenraum der Kartusche 6 evakuiert werden, wenn der Dichtungskolben 20, wie in den Figuren gezeigt, mit dem Sterilisationskolben 22 verbunden ist und der Innenraum der Kartusche 6 dadurch nach außen bis auf die Öffnung zur Vakuumleitung 26 abgedichtet ist.

Gleichzeitig mit der fortwährenden Bewegung des Vakuumkolbens 30 wird über die Kabel 86, 90 oder die Stange 86, 90 und das Kugelgelenk 84 der Zylinder 76 in der Hülse 80 in Längsrichtung bewegt und dabei über die Gewinde 78, 82 gedreht. Die Bewegung in Längsrichtung und die Drehung wird über die Mischwelle 12 beziehungsweise das Kabel 12 durch die Durchführung auf die Mischeinrichtung 10 im Innenraum der Kartusche 6 übertragen. Durch mehrfaches Schwenken des Hebels 4 in beide Richtungen und damit Bewegen der Mischeinrichtung 10 im Innenraum der Kartusche 6 wird der Inhalt, nämlich das Knochenzementpulver 9 und die eingesaugte Monomerflüssigkeit, durchmischt und dadurch ein Knochenzementteig 96 im Innenraum der Kartusche 6 erzeugt.

Wenn die Ausgangskomponenten im Innenraum der Kartusche 6 mit den Mischflügeln 10 gemischt wurden, wird das Kartuschensystem 1 von dem Gehäuse 19 beziehungsweise dem Außengewinde 16 des Gehäuses 19 abgeschraubt und das Kabel 12 beziehungsweise die Mischwelle 12 mit der Mischeinrichtung 10 aus dem Innenraum der Kartusche 6 herausgezogen. Dabei klappen die Mischflügel 10 nach oben zusammen. Hierfür sind an der Verbindung der Mischflügel 10 zu der Mischwelle 12 Material-Verjüngungen als Sollknickstellen vorgesehen.

Der Dichtungskolben 20 wird gegen den Sterilisationskolben 22 gedreht und somit die Gasdurchführung durch den Dichtungskolben 20 verschlossen. Die Vakuumleitung 26 wird vom Dichtungskolben 20 abgezogen. Nachdem das Kartuschensystem 1 abgeschraubt wurde, wird in das Innengewinde 14 ein Austragsrohr (nicht gezeigt) mit einem passenden Außengewinde eingeschraubt, durch das der gemischte Knochenzement 96 appliziert werden kann. Der aus dem Sterilisationskolben 22 und dem Dichtungskolben 20 zusammengesetzte Förderkolben 8 oder Austragskolben 8 wird entrastet und kann mit einem Applikationsgerät (nicht gezeigt) ins Innere der Kartusche 6 getrieben werden. Dadurch wird der Inhalt der Kartusche 6, also der unter Unterdruck gemischte Knochenzementteig 96 aus der gegenüberliegenden Öffnung und durch das aufgeschraubte Austragsrohr ausgepresst.

Die Bauteile der Vakuummischvorrichtung können bis auf die Glasampulle 54, die Filter 64, 72 und die Ausgangskomponenten des Knochenzements durch Spritzgießen aus Kunststoff gefertigt werden. Die Fluidverbindung 70 kann aus einem anderen Kunststoff bestehen. Die Verbindungsleitung 26 kann flexibel sein, um sie leichter von dem Dichtungskolben 20 abziehen zu können.

Die Leitungen 26, 70 und die Kabel 34, 86, 90 oder die gegabelte Stange 34, 86, 90 sind in dem Gehäuse 19 aus Kunststoff angeordnet, das mit dem Fußteil 18 fest verbunden ist, wobei das Fußteil 18 einen flachen Boden aufweist, damit die Vakuummischvorrichtung auf einer flachen Unterlage aufgestellt werden kann.

Anstatt der mit dem beschriebenen Ausführungsbeispiel verwendeten Glasampulle 54 kann auch ein anderer Monomerflüssigkeitsbehälter verwendet werden. Beispielsweise kann ein Folienbeutel enthaltend die Monomerflüssigkeit als Behälter für die Monomerflüssigkeit in eine modifizierte Aufnahme eingesetzt werden. Der Folienbeutel kann beispielsweise ein mit Aluminium beschichteter Kunststoffbeutel sein, der gegen die Monomerflüssigkeit chemisch ausreichend beständig ist. In der alternativen Aufnahme 2 kann dann ein Dorn oder besser eine Klinge vorgesehen sein, der oder die mit der Öffnungseinrichtung 5 gegen den Folienbeutel zu drücken und bewegen ist, so dass der Folienbeutel über die Öffnungseinrichtung 5 mit dem Dorn oder der Klinge aufzustechen oder aufzuschlitzen ist, so dass anschließend die Monomerflüssigkeit aus dem Folienbeutel ausläuft und in der Aufnahme 2 zur Verfügung steht. Ferner kann der Behälter für die Monomerflüssigkeit auch fest in der Aufnahme 2 und damit in die Vakuummischvorrichtung integriert sein und sich mit der Öffnungseinrichtung 5 zu dem Filter 64 und/oder Sieb 64 beziehungsweise zum Druckpumpraum 36 hin öffnen lassen.

Die Variante mit Glasampulle 54 als Behälter für die Monomerflüssigkeit ist aber erfindungsgemäß bevorzugt, da die mit Monomerflüssigkeit gefüllten Glasampullen 54 kommerziell kostengünstig zu erhalten sind und zudem Glasampullen 54 zur langfristigen Lagerung der Monomerflüssigkeit besonders gut geeignet sind. Dabei ist es besonders bevorzugt, dass die Glasampulle 54 bereits in der Aufnahme 2 der Vakuummischvorrichtung enthalten ist.

Statt einer kombinierten Druck-Vakuumpumpe 3, wie mit dem Ausführungsbeispiel nach den Figuren 1 bis 9 gezeigt, können auch eine Druckpumpe und eine zur Druckpumpe separate Vakuumpumpe ohne einen gemeinsamen Hohlzylinder in einer erfindungsgemäßen Vakuummischvorrichtung verwendet werden. Sowohl die Druckpumpe als auch die Vakuumpumpe haben dann beispielsweise jeweils einen eigenen Hohlzylinder und sind über jeweils ein eigenes Kabel oder eine eigene Stange mit einem Rastmittel oder mit der Stange 90 beziehungsweise dem Kabel 90 verbunden. Das Kabel 90 beziehungsweise die Stange 90 spaltet sich dazu dann anstatt in zwei in drei Kabel beziehungsweise Stangen auf, eine für die Druckpumpe, eine für die Vakuumpumpe und eine für den Antrieb der Mischvorrichtung beziehungsweise für die Mischeinrichtung unmittelbar.

Mit der beschriebenen Vakuummischvorrichtung können die beiden Ausgangskomponenten des Knochenzements gelagert und zu einem beliebigen späteren Zeitpunkt unter Vakuum gemischt werden. Dabei muss die Vakuummischvorrichtung an keine externe Versorgung (Strom, Wasser oder Druckgas) angeschlossen werden. Es ist kein interner Energiespeicher, wie eine Batterie, eine Druckgaspatrone oder eine gespannte Feder, zum Antreiben der Vakuummischvorrichtung beziehungsweise der Vakuumpumpe 3, der Druckpumpe 3, der Mischeinrichtung 10 und der Öffnungseinrichtung 5 notwendig. Die zum Erzeugen des Unterdrucks notwendige Energie wird ebenso manuell aufgebracht, wie die zum Öffnen der Glasampulle 54 und die zum Bewegen der Mischeinrichtung 10 notwendige Kraft.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Kartuschensystem
- 2: Aufnahme
- 3: Kombinierte Druck-Vakuumpumpe
- 4: Bedienelement / Hebel
- 5: Öffnungseinrichtung
- 6: Kartusche / Kartuschenwand / Hohlzylinder
- 8: Austragskolben
- 9: Zementpulver
- 10: Mischeinrichtung / Mischflügel
- 12: Mischwelle / Kabel
- 14: Innengewinde
- 16: Außengewinde
- 18: Fußteil / Standfuß
- 19: Gehäuse
- 20: Dichtungskolben
- 22: Sterilisationskolben
- 24: Porenscheibe
- 25: Einmündung
- 26: Verbindungsleitung / Vakuumleitung
- 27: Rückschlagventil
- 28: Rückschlagventil / Abluft
- 29: Hohlzylinder
- 30: Vakuumkolben
- 31: Pumpkolben
- 32: Dichtung / O-Ring
- 33: Verschluss
- 34: Kabel / flexible Stange
- 35: Rohr / Stange / Verbindung
- 36: Druckpumpraum
- 37: Rastmittel
- 38: Gegenrastmittel
- 40: Achse
- 41: Hebelarm
- 42: Hebelarm
- 44: Griff
- 46: Hebel
- 47: Achse
- 48: Kante
- 50: Elastischer Einsatz
- 52: Hohlzylinder
- 54: Glasampulle mit Monomerflüssigkeit
- 56: Ampullenkopf
- 58: Ampullenboden
- 60: Auflage / Absatz
- 62: Hohlzylinder
- 64: Filter / Sieb
- 66: Zwischenraum
- 68: Trichter
- 70: Fluidverbindung / Flüssigkeitsleitung
- 71: Schlaufe
- 72: Pulverundurchlässiger und Flüssigkeitsdurchlässiger Filter
- 73: Ringförmiger Kanal
- 74: Stift / Umlenkrolle
- 76: Zylinder
- 78: Außengewinde / Nocke
- 80: Hülse
- 82: Innengewinde
- 83: Evolvente
- 84: Kugelgelenkkopf
- 86: Kabel / flexible Stange
- 88: Stift / Umlenkrolle
- 90: Kabel / flexible Stange
- 91: Kugel
- 92: Feder
- 94: Vakuumpumpraum
- 96: Knochenzementteig

## Patentansprüche

1. Vakuummischvorrichtung zum Mischen von Polymethylmethacrylat-Knochenzement aus einer Monomerflüssigkeit und einem Zementpulver (9), die Vakuummischvorrichtung aufweisend
zumindest eine Kartusche (6) umfassend einen evakuierbaren Innenraum zum Mischen des Knochenzements (96),
eine Mischeinrichtung (10) zum Durchmischen des Inhalts des Innenraums der zumindest einen Kartusche (6), die beweglich im Innenraum angeordnet ist,
eine Aufnahme (2) zur Aufnahme eines die Monomerflüssigkeit enthaltenden separaten Behälters (54) oder aufweisend einen integrierten Behälter enthaltend die Monomerflüssigkeit,
eine Öffnungseinrichtung (5), die im Bereich der Aufnahme (2) gegen die Aufnahme (2) beweglich angeordnet ist, so dass durch eine Bewegung der Öffnungseinrichtung (5) ein in der Aufnahme (2) angeordneter separater Behälter (54) mit der Öffnungseinrichtung (5) zu öffnen ist, oder die Öffnungseinrichtung (5) im Bereich des integrierten Behälters gegen den integrierten Behälter beweglich angeordnet ist, so dass durch eine Bewegung der Öffnungseinrichtung (5) der integrierte Behälter mit der Öffnungseinrichtung (5) zu öffnen ist,
eine Vakuumpumpe (3), in der ein beweglicher Vakuumkolben (30) zum Erzeugen eines Unterdrucks angeordnet ist und der einen Vakuumpumpraum (94) der Vakuumpumpe (3) begrenzt,
eine Druckpumpe (3), in der ein beweglicher Pumpkolben (31) zum Fördern einer Flüssigkeit angeordnet ist und der einen Druckpumpraum (36) der Druckpumpe (3) begrenzt,
eine Verbindungsleitung (26), die den Innenraum der zumindest einen Kartusche (6) mit dem Vakuumpumpraum (94) der Vakuumpumpe (3) verbindet, und
eine Fluidverbindung (70), die den Innenraum der zumindest einen Kartusche (6) mit dem Druckpumpraum (36) der Druckpumpe (3) verbindet, wobei die Vakuummischvorrichtung ein von außen bedienbares Bedienelement (4) aufweist, wobei mit dem Bedienelement (4) der Vakuumkolben (30) in der Vakuumpumpe (3) manuell bewegbar ist,
mit demselben Bedienelement (4) der Pumpkolben (31) in der Druckpumpe (3) manuell bewegbar ist,
mit demselben Bedienelement (4) die Öffnungseinrichtung (5) gegen die Aufnahme (2) oder gegen den integrierten Behälter zu bewegen ist, und wobei mit demselben Bedienelement (4) die Mischeinrichtung (10) im Innenraum der Kartusche (6) zum Durchmischen des Inhalts des Innenraums der Kartusche (6) bewegbar ist.

2. Vakuummischvorrichtung nach Anspruch 1, **dadurch gekennzeichnet, dass**
das Bedienelement (4) mit dem Vakuumkolben (30) und mit dem Pumpkolben (31) derart verbunden oder verbindbar ist, dass der Vakuumkolben (30) in der Vakuumpumpe (3) und der Pumpkolben (31) in der Druckpumpe (3) durch Bedienen des Bedienelements (4) manuell bewegbar ist.

3. Vakuummischvorrichtung nach einem der vorangehenden Ansprüche, **dadurch gekennzeichnet, dass**
die Öffnungseinrichtung (5) einen ersten Hebel (46) aufweist, der um eine erste Achse (47) drehbar gegen die Aufnahme (2) oder den integrierten Behälter gelagert ist, wobei ein freies Ende (48) des ersten Hebels (46) gegen eine verformbare Seitenwand (50) der Aufnahme (2) oder den integrierten Behälter drückbar ist, wobei das Bedienelement (4) durch einen zweiten Hebel (4) gebildet ist, der um eine zweite Achse (40) drehbar gegen die Aufnahme (2) oder den integrierten Behälter gelagert ist, wobei die zweite Achse (40) den zweiten Hebel (4) in einen kurzen Hebelarm (42) und einen langen Hebelarm (41) teilt, wobei ein Ende des kurzen Hebelarms (42) durch manuelles Bedienen des langen Hebelarms (41) gegen den ersten Hebel (46) zu drücken ist, so dass das freie Ende des ersten Hebels (46) gegen die verformbare Seitenwand (50) drückt und diese derart verformt, dass eine in der Aufnahme (2) befindlicher separater Behälter (54) zu öffnen ist, oder das freie Ende des ersten Hebels (46) gegen den integrierten Behälter drückt, so dass der integrierte Behälter sich zu einer Fluidverbindung (70) hin öffnet.

4. Vakuummischvorrichtung nach einem der vorangehenden Ansprüche, **dadurch gekennzeichnet, dass**
das Bedienelement (4) manuell beweglich ist, vorzugsweise ein um eine Achse (40) schwenkbarer Hebel (4) ist, wobei das Bedienelement (4) derart mit der Öffnungseinrichtung (5), der Vakuumpumpe (3), der Druckpumpe (3) und der Mischeinrichtung (10) in Wirkverbindung steht oder in Wirkverbindung zu bringen ist, dass bei einem ersten Bedienen des Bedienelements (4) ein separater Behälter (54) in der Aufnahme (2) oder der integrierte Behälter zu öffnen ist und bei einem weiteren Bedienen des Bedienelements (4) der Vakuumkolben (30) in der Vakuumpumpe (3) anzutreiben ist, der Pumpkolben (31) in der Druckpumpe (3) anzutreiben ist und die Mischeinrichtung (10) im Innenraum anzutreiben ist.

5. Vakuummischvorrichtung nach Anspruch 4, **dadurch gekennzeichnet, dass**
der Vakuumkolben (30) der Vakuumpumpe (3), der Pumpkolben (31) der Druckpumpe (3) und/oder die Mischeinrichtung (10) über ein flexibles Kabel (34, 86, 90) und/oder eine Stange anzutreiben sind, wobei an dem flexiblen Kabel (34, 86, 90) und/oder der Stange ein Rastmittel (37) vorgesehen ist, das nach erstmaligem Bedienen des Bedienelements (4) in ein Gegenrastmittel (38) am Bedienelement (4) oder in ein mit dem Bedienelement (4) verbundenes Gegenrastmittel (38) greift, so dass bei einem der Rastung nachfolgenden Bedienen des Bedienelements (4) der Vakuumkolben (30) der Vakuumpumpe (3), der Pumpkolben (31) der Druckpumpe (3) und/oder die Mischeinrichtung (10) über das Kabel (34, 86, 90) und/oder die Stange mit dem Bedienelement (4) anzutreiben sind.

6. Vakuummischvorrichtung nach einem der vorangehenden Ansprüche, **dadurch gekennzeichnet, dass**
der Vakuumpumpraum (94) der Vakuumpumpe (3) gasdicht ist und im Inneren der Vakuumpumpe (3) angeordnet ist, wobei der Vakuumkolben (30) über das Bedienelement (4) manuell in zumindest einer Richtung antreibbar ist, so dass durch die Bewegung des Vakuumkolbens (30) der Vakuumpumpraum (94) zu vergrößern ist und mit dem dadurch im Vakuumpumpraum (94) entstehenden Unterdruck der Innenraum der zumindest einen Kartusche (6) durch die Verbindungsleitung (26) evakuierbar ist.

7. Vakuummischvorrichtung nach einem der vorangehenden Ansprüche, **dadurch gekennzeichnet, dass**
der Druckpumpraum (36) der Druckpumpe (3) flüssigkeitsdicht ist und im Inneren der Druckpumpe (3) angeordnet ist, wobei der Pumpkolben (31) über das Bedienelement (4) manuell in zumindest einer Richtung antreibbar ist, so dass durch die Bewegung des Pumpkolbens (31) der Druckpumpraum (36) zu verkleinern ist und mit dem dadurch im Druckpumpraum (36) entstehenden Druck die Monomerflüssigkeit aus dem Druckpumpraum (36) durch die Fluidverbindung (70) in den Innenraum der zumindest einen Kartusche (6) zu drücken ist.

8. Vakuummischvorrichtung nach einem der vorangehenden Ansprüche, **dadurch gekennzeichnet, dass**
der Vakuumkolben (30) und/oder der Pumpkolben (31) über eine Stange (34, 35, 90) und/oder ein Kabel (34, 90) mit dem Bedienelement (4) verbunden oder verbindbar ist oder sind und vorzugsweise der Vakuumkolben (30) durch Bedienen des Bedienelements (4) in der Vakuumpumpe (3) und/oder der Pumpkolben (31) durch Bedienen des Bedienelements (4) in der Druckpumpe (3) zu bewegen ist.

9. Vakuummischvorrichtung nach einem der vorangehenden Ansprüche, **dadurch gekennzeichnet, dass**
in dem Innenraum der Kartusche (6) ein beweglicher Austragskolben (8) zum Austragen des gemischten Knochenzements (96) aus der Kartusche (6) angeordnet ist, wobei der Austragskolben (8) vorzugsweise lösbar arretiert oder arretierbar ist, um eine Bewegung des Austragskolbens (8) unter Einwirkung des Unterdrucks zu verhindern.

10. Vakuummischvorrichtung nach einem der vorangehenden Ansprüche, **dadurch gekennzeichnet, dass**
die Kartusche (6) eine mit Zementpulver (9) gefüllte Zementkartusche (6) ist und in der Aufnahme (2) ein separater Behälter (54) enthaltend eine Monomerflüssigkeit angeordnet ist oder in dem integrierten Behälter eine Monomerflüssigkeit enthalten ist, wobei vorzugsweise die Aufnahme (2) oder der integrierte Behälter durch ein zu öffnendes Trennelement flüssigkeitsundurchlässig mit dem Innenraum der Zementkartusche (6) verbunden ist und/oder der Innenraum der Zementkartusche (6) mit der Vakuumpumpe (3) gasdurchlässig verbunden ist oder verbindbar ist.

11. Vakuummischvorrichtung nach einem der vorangehenden Ansprüche, **dadurch gekennzeichnet, dass**
die Kartusche (6), die Vakuumpumpe (3), die Druckpumpe (3) und alle Leitungen (26, 70) sowie die Aufnahme (2) oder der integrierte Behälter mit einem gemeinsamen Fußteil (18) und/oder einem Gehäuse (19) fest und/oder lösbar verbunden sind, wobei vorzugsweise die Vakuumpumpe (3), die Druckpumpe (3) und alle Leitungen (26, 70) sowie die Aufnahme (2) oder der separate Behälter (54) fest mit dem Fußteil (18) und/oder einem Gehäuse (19) verbunden sind und die Kartusche (6) lösbar mit dem Fußteil (18) und/oder einem Gehäuse (19) verbunden ist.

12. Vakuummischvorrichtung nach einem der vorangehenden Ansprüche, **dadurch gekennzeichnet, dass**
die Vakuumpumpe (3) und die Druckpumpe (3) als kombinierte Druck-Vakuumpumpe (3) aufgebaut ist, die Druck-Vakuumpumpe (3) aufweisend einen Hohlzylinder (29), mit einem gasdichten Verschluss an einem ersten Hohlzylinderende und einen flüssigkeitsdichten Verschluss (33) an dem gegenüberliegenden zweiten Hohlzylinderende, wobei der Hohlzylinder (29) an dem ersten und an dem zweiten Hohlzylinderende mit dem Innenraum der Kartusche (6) verbunden oder verbindbar ist,
den Vakuumkolben (30), der im Hohlzylinder (29) gasdicht, axial beweglich angeordnet ist, und
den Pumpkolben (31), der im Hohlzylinder (29) flüssigkeitsdicht, axial beweglich angeordnet ist, wobei
der Vakuumkolben (30) und der Pumpkolben (31) in der Druck-Vakuumpumpe (3) mit dem manuell bedienbaren Bedienelement (4) bewegbar sind, wobei bei einem Bewegen des Vakuumkolbens (30) mit dem manuell bedienbaren Bedienelement (4) der Vakuumkolben (30) axial vom gasdichten Verschluss weg bewegbar ist und dadurch Gas aus dem Innenraum der Kartusche (6) evakuiert, und
bei einem Bewegen des Pumpkolbens (31) mit dem manuell bedienbaren Bedienelement (4) der Pumpkolben (31) axial zum flüssigkeitsdichten Verschluss (33) hin bewegbar ist und dadurch eine Monomerflüssigkeit aus dem Pumpraum (36) in den Innenraum der Kartusche (6) hinein drückbar ist, wobei
das Bedienelement (4) mit der Öffnungseinrichtung (5) in Wirkverbindung steht und wobei
das Bedienelement (4) mit der Mischeinrichtung (10) im Innenraum der Kartusche (6) derart verbunden ist, dass bei einem Bedienen des Bedienelements (4) die Mischeinrichtung (10) im Innenraum der Kartusche (6) bewegbar ist.

13. Vakuummischvorrichtung nach einem der vorangehenden Ansprüche, **dadurch gekennzeichnet, dass**
die Vakuumpumpe (3), die Druckpumpe (3), die Öffnungseinrichtung (5) und die Mischeinrichtung (10) über die Bewegung des Bedienelements (4) antreibbar sind, wobei bevorzugt die Bewegung des Bedienelements (4) durch manuelle Krafteinwirkung erfolgt.

14. Vakuummischvorrichtung nach einem der vorangehenden Ansprüche, **dadurch gekennzeichnet, dass**
die Vakuumpumpe (3) und die Druckpumpe (3) als kombinierte Druck-Vakuumpumpe (3) ausgeführt sind, wobei vorzugsweise der Vakuumkolben (30) und Pumpkolben (31) miteinander über eine Verbindung (35), insbesondere über eine Stange oder ein Rohr (35), beabstandet sind.

15. Vakuummischvorrichtung nach Anspruch 14, **dadurch gekennzeichnet, dass** der Abstand zwischen den voneinander abgewandten Seiten des Vakuumkolbens (30) und des Pumpkolbens (31) zumindest genauso groß ist wie der maximale Hub des Vakuumkolbens (30) und des Pumpkolbens (31).

16. Vakuummischvorrichtung nach einem der vorangehenden Ansprüche, **dadurch gekennzeichnet, dass**
der Pumpkolben (31) nur in eine Richtung zur Verkleinerung des Druckpumpenraums (36) bewegbar ist und anschließend nicht wieder in die entgegengesetzte Richtung bewegbar ist, vorzugsweise von der Verbindung (35) nur in eine Richtung zur Verkleinerung des Druckpumpenraums bewegbar ist.

17. Verfahren zur Vermischung von Polymethylmethacrylat-Knochenzement in einem Innenraum einer Kartusche (6) einer Vakuummischvorrichtung, insbesondere einer Vakuummischvorrichtung nach einem der vorangehenden Ansprüche, **dadurch gekennzeichnet, dass**
ein Bedienelement (4) bedient wird und dadurch ein integrierter Behälter der Vakuummischvorrichtung oder ein separater Behälter (54), der in einer Aufnahme (2) der Vakuummischvorrichtung angeordnet ist, geöffnet wird, wobei anschließend eine in dem integrierten Behälter oder dem separaten Behälter (54) enthaltene Monomerflüssigkeit als erste Komponente des Knochenzements (96) in einen Druckpumpraum (36) einer Druckpumpe (3) fließt,
durch eine anschließende weitere Bedienung des Bedienelements (4) eine Bewegung eines Vakuumkolbens (30) einer Vakuumpumpe (3) der Vakuummischvorrichtung angetrieben wird, wobei durch die Bewegung des Vakuumkolbens (30) ein Unterdruck in einem Vakuumpumpraum (94) der Vakuumpumpe (3) erzeugt wird, wobei mit der derart angetriebenen Vakuumpumpe (3) der Innenraum der Kartusche (6) evakuiert wird,
durch die weitere Bedienung des Bedienelements (4) eine Bewegung eines Pumpkolbens (31) der Druckpumpe (3) der Vakuummischvorrichtung angetrieben wird, wobei durch die Bewegung des Pumpkolbens (31) ein Druck auf die Monomerflüssigkeit im Druckpumpraum (36) ausgeübt wird und die Monomerflüssigkeit aus dem Druckpumpraum (36) in den Innenraum der Kartusche (6) gedrückt wird, wobei sich in dem Innenraum der Kartusche (6) bereits ein Knochenzementpulver (9) als zweite Komponente des Knochenzements (96) befindet, und
durch die Bedienung des Bedienelements (4) eine Mischeinrichtung (10) im Innenraum der Kartusche (6) bewegt wird und durch die Bewegung der Mischeinrichtung (10) ein Knochenzementteig (96) im Innenraum der Kartusche (6) aus dem Zementpulver (9) und der Monomerflüssigkeit gemischt wird.

18. Verfahren nach Anspruch 17, **dadurch gekennzeichnet, dass**
das Volumen eines Vakuumpumpraums (94) der Vakuumpumpe (3) durch die manuelle Bewegung des Vakuumkolbens (30) vergrößert wird und durch den dadurch entstehenden Unterdruck der Innenraum der Kartusche (6) evakuiert wird und/oder das Volumen des Druckpumpraums (36) der Druckpumpe (3) durch die manuelle Bewegung des Pumpkolbens (31) verkleinert wird und durch den dadurch entstehenden Druck die Monomerflüssigkeit aus dem Druckpumpraum (36) in den Innenraum der Kartusche (6) gedrückt wird.

19. Verfahren nach einem der Ansprüche 17 oder 18, **dadurch gekennzeichnet, dass**
der Vakuumkolben (30) der Vakuumpumpe (3) bewegt wird, wodurch ein Unterdruck gegenüber der umgebenden Atmosphäre in der Vakuumpumpe (3) erzeugt wird,
dabei durch eine Verbindungsleitung (26) Gas aus dem Innenraum der Kartusche (6) in den Vakuumpumpraum (94) der Vakuumpumpe (3) gesaugt wird und
der Pumpkolben (31) der Druckpumpe (3) mit dem Bedienelement (4) bewegt wird, wodurch ein Druck auf die Monomerflüssigkeit in dem Druckpumpraum (36) ausgeübt wird,
die Monomerflüssigkeit durch eine Fluidverbindung (70) vom Druckpumpraum (36) in den Innenraum der Kartusche (6) gedrückt wird,
anschließend durch Bedienen desselben Bedienelements (4) die Mischeinrichtung (10) im Innenraum der Kartusche (6) bewegt wird und dabei das Zementpulver (9) mit der Monomerflüssigkeit vermischt wird, anschließend die Kartusche (6) mit dem gemischten Zementteig (96) entnommen wird und
durch axiale Bewegung eines Austragskolbens (8) der Zementteig (96) aus der Kartusche (6) heraus gepresst wird.

20. Verfahren nach einem der Ansprüche 17 bis 19, **dadurch gekennzeichnet, dass**
das Zementpulver (9) in der Kartusche (6) angeordnet ist,
die Monomerflüssigkeit in einer von der Kartusche (6) separaten Aufnahme (2) angeordnet ist, wobei die Monomerflüssigkeit in einem integrierten Behälter oder in einem separaten Behälter (54), vorzugsweise in einer Glasampulle (54) in der Aufnahme (2), enthalten ist,
der integrierte Behälter oder der separate Behälter (54) durch Bedienen des Bedienelements (4) und einer daraus resultierenden Bewegung der Öffnungseinrichtung (5) geöffnet wird, bevor der Vakuumkolben (30) und der Pumpkolben (31) durch ein weiteres Bedienen des Bedienelements (4) angetrieben werden,
danach der Vakuumkolben (30) und der Pumpkolben (31) axial in einem Hohlzylinder (29) bewegt werden,
dabei durch die Verbindungsleitung (26) Gas aus dem Innenraum der Kartusche (6) in den durch den Hohlzylinder (29) begrenzten Vakuumpumpraum (94) gesaugt wird und die in dem durch den Hohlzylinder (29) begrenzten Druckpumpraum (36) befindliche Monomerflüssigkeit durch die Fluidverbindung (70) in den Innenraum der Kartusche (6) gedrückt wird.

## Claims

1. Vacuum mixing device for the mixing of polymethylmethacrylate bone cement from a monomer liquid and a cement powder (9), whereby the vacuum mixing device comprises
at least one cartridge (6) comprising an internal space, which can be evacuated, for the mixing of the bone cement (96);
a mixing facility (10) for mixing the content of the internal space of the at least one cartridge (6) that is arranged such as to be mobile in the internal space; a receptacle (2) for accommodating a separate container (54) that contains the monomer liquid or comprising an integrated container containing the monomer liquid;
an opening facility (5) that is arranged in the area of the receptacle (2) such as to be mobile with respect to the receptacle (2) such that a motion of the opening facility (5) allows a separate container (54) arranged in the receptacle (2) to be opened by the opening facility (5), or the opening facility (5) is arranged in the area of the integrated container such as to be mobile with respect to the integrated container such that a motion of the opening facility (5) allows the integrated container to be opened by the opening facility (5);
a vacuum pump (3), in which a mobile vacuum plunger (30) for generation of a negative pressure is arranged and which limits a vacuum pump space (94) of the vacuum pump (3);
a pressure pump (3), in which a mobile pump plunger (31) for conveying a liquid is arranged and limits a pressure pump space (36) of the pressure pump (3);
a connecting line (26) that connects the internal space of the at least one cartridge (6) to the vacuum pump space (94) of the vacuum pump (3); and
a fluid connection (70) that connects the internal space of the at least one cartridge (6) to the pressure pump space (36) of the pressure pump (3), whereby the vacuum mixing device comprises an operating element (4) that can be operated from outside, whereby the vacuum plunger (30) can be moved manually in the vacuum pump (3) by means of the operating element (4);
the pump plunger (31) in the pressure pump (3) can be moved manually by means of the same operating element (4);
the opening facility (5) can be moved against the receptacle (2) or against the integrated container by means of the same operating element (4), and whereby the mixing facility (10) can be moved in the internal space of the cartridge (6) by means of the same operating element (4) in order to mix the content of the internal space of the cartridge (6).

2. Vacuum mixing device according to claim 1, **characterised in that** the operating element (4) is or can be connected appropriately to the vacuum plunger (30) and to the pump plunger (31) such that the vacuum plunger (30) can be moved manually in the vacuum pump (3) and the pump plunger (31) can be moved manually in the pressure pump (3) by operating the operating element (4).

3. Vacuum mixing device according to any one of the preceding claims, **characterised in that**
the opening facility (5) comprises a first lever (46) that is supported against the receptacle (2) or the integrated container such that it can rotate about a first axis (47), whereby a free end (48) of the first lever (46) can be pushed against a deformable side wall (50) of the receptacle (2) or the integrated container, whereby the operating element (4) is formed by a second lever (4) that is supported against the receptacle (2) or the integrated container such that it can rotate about a second axis (40), whereby the second axis (40) divides the second lever (4) into a short lever arm (42) and a long lever arm (41), whereby one end of the short lever arm (42) can be pushed against the first lever (46) by manual operation of the long lever arm (41) such that the free end of the first lever (46) pushes against the deformable side wall (50) and deforms said side wall appropriately such that a separate container (54) that is situated in the receptacle (2) can be opened, or the free end of the first lever (46) pushes against the integrated container such that the integrated container opens toward a fluid connection (70).

4. Vacuum mixing device according to any one of the preceding claims,
**characterised in that**
the operating element (4) can be moved manually, preferably it is a lever (4) that can be swivelled about an axis (40), whereby the operating element (4) is or can be brought into an operative connection to the opening facility (5), the vacuum pump (3), the pressure pump (3), and the mixing facility (10) in appropriate manner such that a separate container (54) in the receptacle (2) or the integrated container can be opened by means of a first operation of the operating element (4), and, by means of another operation of the operating element (4), the vacuum plunger (30) can be driven in the vacuum pump (3), the pump plunger (31) can be driven in the pressure pump (3), and the mixing facility (10) can be driven in the internal space.

5. Vacuum mixing device according to claim 4, **characterised in that**
the vacuum plunger (30) of the vacuum pump (3), the pump plunger (31) of the pressure pump (3) and/or the mixing facility (10) can be driven via a flexible cable (34, 86, 90) and/or a rod, whereby the flexible cable (34, 86, 90) and/or the rod have a snap-in means (37) provided on them, which, after the operating element (4) is operated for the first time, engages an opposite snap-in means (38) on the operating element (4) or an opposite snap-in means (38) that is connected to the operating element (4) such that an operation of the operating element (4) after the snapping-in has taken place can drive the vacuum plunger (30) of the vacuum pump (3), the pump plunger (31) of the pressure pump (3) and/or the mixing facility (10) via the cable (34, 86, 90) and/or the rod by means of the operating element (4).

6. Vacuum mixing device according to any one of the preceding claims,
**characterised in that**
the vacuum pump space (94) of the vacuum pump (3) is gas-tight and is arranged on the inside of the vacuum pump (3), whereby the vacuum plunger (30) can be driven manually in at least one direction by means of the operating element (4) such that the motion of the vacuum plunger (30) allows the vacuum pump space (94) to be increased and the negative pressure thus generated in the vacuum pump space (94) allows the internal space of the at least one cartridge (6) to be evacuated through the connecting line (26).

7. Vacuum mixing device according to any one of the preceding claims,
**characterised in that**
the pressure pump space (36) of the pressure pump (3) is liquid-tight and is arranged on the inside of the pressure pump (3), whereby the pump plunger (31) can be manually driven in at least one direction by means of the operating element (4) such that the motion of the pump plunger (31) allows the pressure pump space (36) to be decreased and the pressure thus generated in the pressure pump space (36) allows the monomer liquid to be pushed from the pressure pump space (36) through the fluid connection (70) into the internal space of the at least one cartridge (6).

8. Vacuum mixing device according to any one of the preceding claims,
**characterised in that**
the vacuum plunger (30) and/or the pump plunger (31) is or can be connected to the operating element (4) by means of a rod (34, 35, 90) and/or a cable (34, 90), and, preferably, the vacuum plunger (30) can be moved in the vacuum pump (3) by operation of the operating element (4) and/or the pump plunger (31) can be moved in the pressure pump (3) by operation of the operating element (4).

9. Vacuum mixing device according to any one of the preceding claims,
**characterised in that**
the internal space of the cartridge (6) has a mobile dispensing plunger (8) for dispensing the mixed bone cement (96) from the cartridge (6) arranged in it, whereby the dispensing plunger (8) preferably is or can be locked in detachable manner in order to prevent a motion of the dispensing plunger (8) in response to the action of the negative pressure.

10. Vacuum mixing device according to any one of the preceding claims,
**characterised in that**
the cartridge (6) is a cement powder (9)-filled cement cartridge (6) and a separate container (54) containing a monomer liquid is arranged in the receptacle (2) or a monomer liquid is contained in the integrated container, whereby, preferably, the receptacle (2) or the integrated container is connected in liquid-impermeable manner to the internal space of the cement cartridge (6) by means of a separating element that can be opened and/or the internal space of the cement cartridge (6) is or can be connected to the vacuum pump (3) in gas-permeable manner.

11. Vacuum mixing device according to any one of the preceding claims,
**characterised in that**
the cartridge (6), the vacuum pump (3), the pressure pump (3), and all lines (26, 70) as well as the receptacle (2) or the integrated container are connected, firmly and/or detachably, to a common foot part (18) and/or a casing (19), whereby, preferably, the vacuum pump (3), the pressure pump (3), and all lines (26, 70) as well as the receptacle (2) or the separate container (54) are firmly connected to the foot part (18) and/or a casing (19), and the cartridge (6) is detachably connected to the foot part (18) and/or a casing (19).

12. Vacuum mixing device according to any one of the preceding claims,
**characterised in that**
the vacuum pump (3) and the pressure pump (3) are designed as a combined pressure-vacuum pump (3), the pressure-vacuum pump (3) comprising a hollow cylinder (29) with a gas-tight closure on a first hollow cylinder end and a liquid-tight closure (33) on the opposite second hollow cylinder end, whereby the hollow cylinder (29) is or can be connected to the internal space of the cartridge (6) on the first and on the second hollow cylinder end;
the vacuum plunger (30) that is arranged in the hollow cylinder (29)such as to be gas-tight, axially mobile; and
the pump plunger (31) that is arranged in the hollow cylinder (29) such as to be liquid-tight, axially mobile, whereby
the vacuum plunger (30) and the pump plunger (31) can be moved in the pressure-vacuum pump (3) by means of the manually operable operating element (4), whereby
upon a motion of the vacuum plunger (30) effected by the manually operable operating element (4), the vacuum plunger (30) can be moved away axially from the gas-tight closure and thereby gas is evacuated from the internal space of the cartridge (6), and
upon a motion of the pump plunger (31) effected by the manually operable operating element (4), the pump plunger (31) can be moved axially toward the liquid-tight closure (33) and thereby a monomer liquid can be pushed from the pump space (36) into the internal space of the cartridge (6); whereby the operating element (4) is in operative connection with the opening facility (5); and whereby
the operating element (4) is appropriately connected to the mixing facility (10) in the internal space of the cartridge (6) such that the mixing facility (10) can be moved in the internal space of the cartridge (6) upon an operation of the operating element (4).

13. Vacuum mixing device according to any one of the preceding claims,
**characterised in that**
the vacuum pump (3), the pressure pump (3), the opening facility (5), and the mixing facility (10) can be driven by the motion of the operating element (4), whereby the motion of the operating element (4) preferably takes place by action of a manual force.

14. Vacuum mixing device according to any one of the preceding claims,
**characterised in that**
the vacuum pump (3) and the pressure pump (3) are designed as a combined pressure-vacuum pump (3), whereby, preferably, the vacuum plunger (30) and the pump plunger (31) are kept at a distance from each other by means of a connection (35), in particular by means of a rod or a tube (35).

15. Vacuum mixing device according to claim 14, **characterised in that** the distance between the sides of the vacuum plunger (30) and of the pump plunger (31) that face away from each other is at least equal to the maximum stroke of the vacuum plunger (30) and of the pump plunger (31).

16. Vacuum mixing device according to any one of the preceding claims,
**characterised in that**
the pump plunger (31) can be moved only in one direction for decreasing the pressure pump space (36) and subsequently cannot be moved in the opposite direction, preferably can be moved by the connection (35) only in one direction for decreasing the pressure pump space.

17. Method for the mixing of polymethylmethacrylate bone cement in an internal space of a cartridge (6) of a vacuum mixing device, in particular of a vacuum mixing device according to any one of the preceding claims, **characterised in that**
an operating element (4) is being operated and thereby an integrated container of the vacuum mixing device or a separate container (54), which is arranged in a receptacle (2) of the vacuum mixing device, is being opened, whereby a monomer liquid that is contained in the integrated container or the separate container (54) then flows into a pressure pump space (36) of a pressure pump (3) as first component of the bone cement (96);
a subsequent further operation of the operating element (4) drives a motion of a vacuum plunger (30) of a vacuum pump (3) of the vacuum mixing device, whereby the motion of the vacuum plunger (30) generates a negative pressure in the vacuum pump space (94) of the vacuum pump (3), whereby the internal space of the cartridge (6) is being evacuated by the vacuum pump (3) driven in this manner;
the further operation of the operating element (4) drives a motion of a pump plunger (31) of the pressure pump (3) of the vacuum mixing device, whereby the motion of the pump plunger (31) exerts a pressure onto the monomer liquid in the pressure pump space (36) and pushes the monomer liquid from the pressure pump space (36) into the internal space of the cartridge (6), whereby a bone cement powder (9) is already present in the internal space of the cartridge (6) as second component of the bone cement (96); and
the operation of the operating element (4) moves a mixing facility (10) in the internal space of the cartridge (6), and the motion of the mixing facility (10) mixes a bone cement dough (96) in the internal space of the cartridge (6) from the cement powder (9) and the monomer liquid.

18. Method according to claim 17, **characterised in that**
the volume of a vacuum pump space (94) of the vacuum pump (3) is increased by the manual motion of the vacuum plunger (30) and the negative pressure thus generated evacuates the internal space of the cartridge (6) and/or the volume of the pressure pump space (36) of the pressure pump (3) is decreased by the manual motion of the pump plunger (31) and the pressure thus generated pushes the monomer liquid from the pressure pump space (36) into the internal space of the cartridge (6).

19. Method according to any one of the claims 17 or 18, **characterised in that**
the vacuum plunger (30) of the vacuum pump (3) is being moved, by means of which a negative pressure with respect to the ambient atmosphere is generated in the vacuum pump (3);
in the process, gas from the internal space of the cartridge (6) is aspirated through a connection line (26) into the vacuum pump space (94) of the vacuum pump (3); and
the pump plunger (31) of the pressure pump (3) is moved by means of the operating element (4), by means of which a pressure is exerted onto the monomer liquid in the pressure pump space (36);
the monomer liquid is pushed from the pressure pump space (36) through a fluid connection (70) into the internal space of the cartridge (6);
then the mixing facility (10) is moved in the internal space of the cartridge (6) by operation of the same operating element (4) and, in the process, the cement powder (9) is being mixed with the monomer liquid;
then the cartridge (6) containing the mixed cement dough (96) is being removed; and
the cement dough (96) is being pressed from the cartridge (6) by axial motion of a dispensing plunger (8).

20. Method according to any one of the claims 17 to 19, **characterised in that**
the cement powder (9) is arranged in the cartridge (6);
the monomer liquid is arranged in a receptacle (2) that is separate from the cartridge (6), whereby the monomer liquid is contained in an integrated container or in a separate container (54), preferably in a glass ampoule (54) in the receptacle (2);
the integrated container or the separate container (54) is being opened by operation of the operating element (4) and an ensuing motion of the opening facility (5) before the vacuum plunger (30) and the pump plunger (31) are driven by means of a further operation of the operating element (4); then the vacuum plunger (30) and the pump plunger (31) are moved axially in a hollow cylinder (29);
in the process, gas from the internal space of the cartridge (6) is aspirated through the connecting line (26) into the vacuum pump space (94) that is limited by the hollow cylinder (29), and the monomer liquid that is present in the pressure pump space (36) that is limited by the hollow cylinder (29) is being pushed through the fluid connection (70) into the internal space of the cartridge (6).

## Revendications

1. Dispositif de mélange sous-vide pour mélanger du ciment osseux à base de polyméthacrylate de méthyle à partir d'un monomère liquide et d'une poudre de ciment (9), le dispositif de mélange sous-vide présentant
au moins une cartouche (6) comprenant un espace intérieur pouvant être évacué pour mélanger le ciment osseux (96),
un dispositif de mélange (10) pour mélanger le contenu de l'intérieur de l'au moins une cartouche (6) qui est disposée mobile dans l'espace intérieur,
un logement (2) pour recevoir un récipient séparé (54) contenant le monomère liquide ou présentant un récipient intégré contenant le monomère liquide,
un dispositif d'ouverture (5) qui est disposé au niveau du logement (2) mobile contre le logement (2), de sorte que par un déplacement du dispositif d'ouverture (5), un récipient séparé (54) disposé dans le logement (2) est à ouvrir avec le dispositif d'ouverture (5) ou bien le dispositif d'ouverture (5) est disposé mobile contre le récipient intégré au niveau du récipient intégré, de sorte que par un déplacement du dispositif d'ouverture (5), le récipient intégré est à ouvrir avec le dispositif d'ouverture (5),
un pompe à vide (3) dans laquelle un piston à vide (30) mobile est disposé pour produire une dépression et qui limite une chambre de pompage à vide (94) de la pompe à vide (3),
une pompe à refoulement (3) dans laquelle un piston de pompage (31) mobile pour transporter un liquide est disposé et qui limite un espace de pompe à refoulement (36) de la pompe à refoulement (3),
une conduite de liaison (26) qui relie l'intérieur de l'au moins une cartouche (6) à la chambre de pompage à vide (94) de la pompe à vide (3), et
une communication fluidique (70) qui relie l'intérieur de l'au moins une cartouche (6) à l'espace de pompe à refoulement (36) de la pompe à refoulement (3), dans lequel
le dispositif de mélange sous-vide présente un élément de commande (4) pouvant être commandé par l'extérieur, dans lequel le piston à vide (30) peut être déplacé manuellement dans la pompe à vide (3) avec l'élément de commande (4),
avec ce même élément de commande (4), le piston de pompage (31) peut être déplacé manuellement dans la pompe à refoulement (3),
avec ce même élément de commande (4), le dispositif d'ouverture (5) est à déplacer contre le logement (2) ou contre le récipient intégré, et dans lequel avec ce même élément de commande (4), le dispositif de mélange (10) peut être déplacé à l'espace intérieur de la cartouche (6) pour mélanger le contenu de l'intérieur de la cartouche (6).

2. Dispositif de mélange sous-vide selon la revendication 1, **caractérisé en ce que** l'élément de commande (4) est ainsi relié ou peut être relié au piston à vide (30) et au piston de pompage (31) que le piston à vide (30) peut être déplacé manuellement dans la pompe à vide (30) et le piston de pompage (31) peut être déplacé manuellement dans la pompe à refoulement (3) par la commande de l'élément de commande (4).

3. Dispositif de mélange sous-vide selon l'une des revendications précédentes, **caractérisé en ce que**
le dispositif d'ouverture (5) présente un premier levier (46) qui est disposé rotatif contre le logement (2) sur un premier axe (47) ou autour du récipient intégré, dans lequel une extrémité libre (48) du premier levier (46) peut être appuyée contre une paroi latérale (50) déformable du logement (2) ou le récipient intégré, dans lequel l'élément de commande (4) est formé par un second levier (4) qui est disposé rotatif contre le logement (2) sur un second axe (40) ou autour du récipient intégré, dans lequel le second axe (40) partage le second levier (4) en un bras de levier court (42) et un bras de levier long (41), dans lequel une extrémité du bras de levier court (42) est à appuyer contre le premier levier (46) par une commande manuelle du bras de levier long (41), de sorte que l'extrémité libre du premier levier (46) appuie contre la paroi latérale (50) déformable et déforme celle-ci de telle façon qu'un récipient séparé (54) se trouvant dans le logement (2) est à ouvrir, ou que l'extrémité libre du premier levier (46) appuie contre le récipient intégré de sorte que le récipient intégré s'ouvre en direction d'une communication fluidique (70).

4. Dispositif de mélange sous-vide selon l'une des revendications précédentes, **caractérisé en ce que**
l'élément de commande (4) est mobile manuellement, est de préférence un levier (4) pivotant sur un axe (40), dans lequel l'élément de commande (4) est ainsi en liaison active ou à amener en liaison active avec le dispositif d'ouverture (5), la pompe à vide (3), la pompe à refoulement (3) et le dispositif de mélange (10) que lors d'une première commande de l'élément de commande (4), un récipient séparé (54) dans le logement (2) ou le récipient intégré est à ouvrir et lors d'une commande ultérieure de l'élément de commande (4), le piston à vide (30) dans la pompe à vide (3) est à actionner, le piston de pompage (31) dans la pompe à refoulement (3) est à actionner et le dispositif de mélange (10) dans l'intérieur est à actionner.

5. Dispositif de mélange sous-vide selon la revendication 4, **caractérisé en ce que** le piston à vide (30) de la pompe à vide (3), le piston de pompage (31) de la pompe à refoulement (3) et/ou le dispositif de mélange (10) sont à actionner par le biais d'un câble flexible (34, 86, 90) et/ou d'une barre, dans lequel un moyen de crantage (37) est prévu sur le câble flexible (34, 86, 90) et/ou la barre, qui se met en prise dans un contre-moyen de crantage (38) sur l'élément de commande (4) ou dans un contre-moyen de crantage (38) relié à l'élément de commande (4) après la première utilisation de l'élément de commande (4), de sorte que lors d'une commande de l'élément de commande (4) consécutive au crantage, le piston à vide (30) de la pompe à vide (3), le piston de pompage (31) de la pompe à refoulement (3) et/ou le dispositif de mélange (10) sont à actionner avec l'élément de commande (4) par le biais du câble flexible (34, 86, 90) et/ou de la barre.

6. Dispositif de mélange sous-vide selon l'une des revendications précédentes, **caractérisé en ce que** la chambre de pompage à vide (94) de la pompe à vide (3) est étanche aux gaz et disposé à l'intérieur de la pompe à vide (3), dans lequel le piston à vide (30) peut être actionné dans au moins un sens manuellement par le biais de l'élément de commande (4), de sorte que par le déplacement du piston à vide (30), la chambre de pompage à vide (94) est à agrandir et avec la dépression se produisant ainsi dans la chambre de pompage à vide (94), l'intérieur de l'au moins une cartouche (6) peut être évacué par la conduite de liaison (26).

7. Dispositif de mélange sous-vide selon l'une des revendications précédentes, **caractérisé en ce que** l'espace de pompe à refoulement (36) de la pompe à refoulement (3) est étanche aux liquides et est disposé à l'intérieur de la pompe à refoulement (3), dans lequel le piston de pompage (31) peut être actionné dans au moins un sens manuellement par le biais de l'élément de commande (4), de sorte que par le déplacement du piston de pompage (31), l'espace de pompe à refoulement (36) est à réduire et avec la pression se produisant ainsi dans l'espace de pompe à refoulement (36), le monomère liquide est à comprimer hors de l'espace de pompe à refoulement (36) jusque dans l'intérieur de l'au moins une cartouche (6) par la communication fluidique (70).

8. Dispositif de mélange sous-vide selon l'une des revendications précédentes, **caractérisé en ce que** le piston à vide (30) et/ou le piston de pompage (31) est/sont reliés ou peu(ven)t être relié(s) à l'élément de commande (4) par une barre (34, 35, 90) et/ou un câble (34, 90), et de préférence, le piston à vide (30) est à déplacer dans la pompe à refoulement (3) par commande de l'élément de commande (4) et/ou le piston de pompage (31) est à déplacer dans la pompe à refoulement (3) par commande de l'élément de commande (4).

9. Dispositif de mélange sous-vide selon l'une des revendications précédentes, **caractérisé en ce qu'**un piston d'évacuation (8) mobile pour évacuer le ciment osseux (96) mélangé hors de la cartouche (6) est disposé dans l'intérieur de la cartouche (6), dans lequel le piston d'évacuation (8) est bloqué ou peut être bloqué de manière amovible de préférence pour empêcher un déplacement du piston d'évacuation (8) sous l'effet de la dépression.

10. Dispositif de mélange sous-vide selon l'une des revendications précédentes, **caractérisé en ce que** la cartouche (6) est une cartouche de ciment (6) remplie de poudre de ciment (9) et dans le logement (2), un récipient séparé (54) contenant un monomère liquide est disposé ou bien un monomère liquide est contenu dans le récipient intégré, dans lequel de préférence, le logement (2) ou le récipient intégré est relié sans laisser passer de liquides avec l'intérieur de la cartouche de ciment (6) par un élément de séparation à ouvrir et/ou l'intérieur de la cartouche de ciment (6) est relié ou être relié à la pompe à vide (3) en laissant passer les gaz.

11. Dispositif de mélange sous-vide selon l'une des revendications précédentes, **caractérisé en ce que** la cartouche (6), la pompe à vide (3), la pompe à refoulement (3) et toutes les conduites (26, 70) ainsi que le logement (2) ou le récipient intégré sont reliés de façon amovible à une partie de pied (18) et/ou un boîtier (19) commun(e), dans lequel de préférence, la pompe à vide (3), la pompe à refoulement (3) et toutes les conduites (26, 70) ainsi que le logement (2) ou le récipient séparé (54) sont reliés fixement à la partie de pied (18) et/ou à un boîtier (19) et la cartouche (6) est reliée de façon amovible à la partie de pied (18) et/ou à un boîtier (19).

12. Dispositif de mélange sous-vide selon l'une des revendications précédentes, **caractérisé en ce que** la pompe à vide (3) et la pompe à refoulement (3) sont montées en tant que pompe à vide-refoulement combinée (3), la pompe à vide-refoulement (3) présentant
un cylindre creux (29) avec une fermeture étanche aux gaz sur une première extrémité de cylindre creux et un élément de fermeture (33) étanche aux liquides sur la seconde extrémité de cylindre creux opposée, dans lequel le cylindre creux (29) est relié ou peut être relié avec l'intérieur de la cartouche (6) sur la première ou la seconde extrémité de cylindre creux,
le piston à vide (30) qui est disposé étanche aux gaz dans le cylindre creux (29) en étant mobile axialement, et
le piston de pompage (31) qui est disposé étanche aux liquides dans le cylindre creux (29), en étant mobile axialement, dans lequel le piston à vide (30) et le piston de pompage (31) sont mobiles dans la pompe à vide-refoulement (3) avec l'élément de commande (4) pouvant être commandé manuellement, dans lequel
lors d'un déplacement du piston à vide (30) avec l'élément de commande (4) pouvant être commandé manuellement, le piston à vide (30) peut être éloigné axialement de la fermeture étanche aux gaz et du gaz peut être ainsi évacué de l'intérieur de la cartouche (6), et
lors d'un déplacement du piston de pompage (31) avec l'élément de commande (4) pouvant être commandé manuellement, le piston de pompage (31) est mobile axialement en direction de la fermeture (33) étanche aux liquides et ainsi un monomère liquide peut être comprimé hors de l'espace de pompe à refoulement (36) jusque dans l'intérieur de la cartouche (6), dans lequel
l'élément de commande (4) est en liaison active avec le dispositif d'ouverture (5) et dans lequel
l'élément de commande (4) est ainsi relié au dispositif de mélange (10) dans l'intérieur de la cartouche (6) que lors d'une commande de l'élément de commande (4), le dispositif de mélange (10) est mobile dans l'intérieur de la cartouche (6).

13. Dispositif de mélange sous-vide selon l'une des revendications précédentes, **caractérisé en ce que** la pompe à vide (3), la pompe à refoulement (3), le dispositif d'ouverture (5) et le dispositif de mélange (10) peuvent être actionnés par le déplacement de l'élément de commande (4), dans lequel de préférence, le déplacement de l'élément de commande (4) s'effectue par action d'une force manuelle.

14. Dispositif de mélange sous-vide selon l'une des revendications précédentes, **caractérisé en ce que** la pompe à vide (3) et la pompe à refoulement (3) sont conçues en tant que pompe à vide-refoulement combinée (3), dans lequel de préférence le piston à vide (30) et le piston de pompage (31) sont distants entre eux par le biais d'une liaison (35), en particulier par une barre ou un tube (35).

15. Dispositif de mélange sous-vide selon la revendication 14, **caractérisé en ce que** la distance entre les côtés détournés l'un de l'autre du piston à vide (30) et du piston de pompage (31) est au moins aussi grande que la course maximale du piston à vide (30) et du piston de pompage (31).

16. Dispositif de mélange sous-vide selon l'une des revendications précédentes, **caractérisé en ce que** le piston de pompage (31) est mobile uniquement dans une direction pour réduire l'espace de pompe à refoulement (36) et n'est ensuite pas mobile de nouveau dans le sens opposé, est de préférence mobile seulement dans un sens depuis la liaison (35) pour réduire l'espace de pompe à refoulement.

17. Procédé destiné à mélanger du ciment osseux à base de polyméthacrylate de méthyle dans un espace intérieur d'une cartouche (6) d'un dispositif de mélange sous-vide, en particulier un dispositif de mélange sous-vide selon l'une des revendications précédentes, **caractérisé en ce**
**qu'**un élément de commande (4) est commandé et qu'ainsi un récipient intégré du dispositif de mélange sous-vide ou un récipient séparé (54) qui est disposé dans un logement (2) du dispositif de mélange sous-vide est ouvert, dans lequel ensuite, un monomère liquide contenu dans le récipient intégré ou le récipient séparé (54) s'écoule en tant que première composante du ciment osseux (96) dans un espace de pompe à refoulement (36) d'une pompe à refoulement (3),
par un déplacement ultérieur consécutif de l'élément de commande (4), un déplacement d'un piston à vide (30) d'une pompe à vide (3) du dispositif de mélange sous-vide est actionné, dans lequel, par le déplacement du piston à vide (30), une dépression est produite dans une chambre de pompage à vide (94) de la pompe à vide (3), dans lequel l'intérieur de la cartouche (6) est évacué avec la pompe à vide (3) ainsi actionnée,
par le déplacement ultérieur de l'élément de commande (4), un déplacement d'un piston de pompage (31) de la pompe à refoulement (3) du dispositif de mélange sous-vide est actionné, dans lequel, par le déplacement du piston de pompage (31), une pression sur le monomère liquide dans l'espace de pompe à refoulement (36) est exercée et le monomère liquide est comprimé hors de l'espace de pompe à refoulement (36) dans l'intérieur de la cartouche (6), dans lequel une poudre de ciment osseux (9) se trouve déjà à l'espace intérieur de la cartouche (6) en tant que seconde composante du ciment osseux (96), et
par la commande de l'élément de commande (4), un dispositif de mélange (10) est déplacé dans l'intérieur de la cartouche (6) et par le déplacement du dispositif de mélange (10), une pâte de ciment osseux (96) est mélangée à l'espace intérieur de la cartouche (6) à partir de la poudre de ciment (9) et du monomère liquide.

18. Procédé selon la revendication 17, **caractérisé en ce que**
le volume d'une chambre de pompage à vide (94) de la pompe à vide (3) est agrandi par le déplacement manuel du piston à vide (30) et par la dépression se produisant ainsi, l'intérieur de la cartouche (6) est évacué et/ou le volume de l'espace de pompe à refoulement (36) de la pompe à refoulement (3) est réduit par le déplacement manuel du piston de pompage (31) et par la pression se produisant ainsi, le monomère liquide est comprimé hors de l'espace de pompe à refoulement (36) jusque dans l'intérieur de la cartouche (6).

19. Procédé selon la revendication 17 ou 18, **caractérisé en ce que**
le piston à vide (30) de la pompe à vide (3) est déplacé, ce par quoi une dépression est produite dans la pompe à vide (3) par rapport à l'atmosphère environnante,
du gaz est ainsi aspiré hors de l'intérieur de la cartouche (6) jusque dans la chambre de pompage à vide (94) de la pompe à vide (3) par une conduite de liaison (26) et
le piston de pompage (31) de la pompe à refoulement (3) est déplacé avec l'élément de commande (4), ce qui fait qu'une pression est exercée sur le monomère liquide dans l'espace de pompe à refoulement (36),
le monomère liquide est comprimé de l'espace de pompe à refoulement (36) jusque dans jusque l'intérieur de la cartouche (6) par une communication fluidique (70),
ensuite, par une commande de ce même élément de commande (4), le dispositif de mélange (10) est déplacé dans l'intérieur de la cartouche (6) et la poudre de ciment (9) est ainsi mélangée au monomère liquide,
ensuite, la cartouche (6) avec la pâte de ciment (96) mélangée est prélevée et par un déplacement axial d'un piston d'évacuation (8), la pâte de ciment (96) est comprimée hors de la cartouche (6).

20. Procédé selon l'une des revendications 17 à 19, **caractérisé en ce que** la poudre de ciment (9) est disposée dans la cartouche (6),
le monomère liquide est disposé dans un logement (2) séparé de la cartouche (6), dans lequel le monomère liquide est contenu dans un récipient intégré ou dans un récipient séparé (54), de préférence dans une ampoule en verre (54) dans le logement (2),
le récipient intégré ou le récipient séparé (54) est ouvert par commande de l'élément de commande (4) et un déplacement qui en résulte du dispositif d'ouverture (5), avant que le piston à vide (30) et le piston de pompage (31) ne soient actionnés par une commande ultérieure de l'élément de commande (4),
ensuite, le piston à vide (30) et le piston de pompage (31) sont déplacés axialement dans un cylindre creux (29),
du gaz est ainsi aspiré hors de l'intérieur de la cartouche (6) jusque dans la chambre de pompage à vide (94) délimité par le cylindre creux (29) par la conduite de liaison (26) et le monomère liquide se trouvant dans l'espace de pompe à refoulement (36) délimité par le cylindre creux (29) est comprimé jusque dans l'intérieur de la cartouche (6) par la communication fluidique (70).
